# EUROPEAN PATENT APPLICATION

(11) **EP 4 729 620 A1**
(43) Date of publication of application: **22.04.2026**
(21) Application number: 24858569.7
(22) Date of filing: 27.08.2024
(51) Int. Cl.: C12N 15/864, C12N 9/02, C12N 9/78, C12N 9/88, C12N 15/53, C12N 15/55, C12N 15/60, C07K 14/015, A61K 48/00, A61P 25/16

(54) **GENE THERAPY VECTOR FOR TREATING PARKINSON'S DISEASE AND USE THEREOF**

(30) Priority: 28.08.2023 CN 202311094965
(71) Applicant: Kanglin Biotech (Hangzhou) Co., Ltd., Hangzhou, Zhejiang 310018 (CN)
(72) Inventor: WU, Haoquan, Zhejiang 310018 (CN); DING, Yanfu, Zhejiang 310018 (CN); SU, Lingling, Zhejiang 310018 (CN)
(74) Representative: Maiwald GmbH
(86) International application number: PCT/CN2024/114821
(87) International publication number: WO 2025/045033

(57) **Abstract**

Provided are a gene therapy vector for treating Parkinson's disease and a use thereof. Specifically, provided is an adeno-associated virus (AAV) vector for treating Parkinson's disease, which can simultaneously express functional tyrosine hydroxylase (TH), GTP-cyclohydrolase 1 (GCH1) and aromatic amino acid decarboxylase (AADC) to promote dopamine synthesis. Also provided are an AAV virus particle containing the AAV vector, a composition containing the AAV vector or the AAV virus particle, and uses of the AAV vector, the AAV virus particle and the composition in the preparation of drugs for preventing or treating Parkinson's disease.

## Description

### Technical Field

The present disclosure relates to the field of gene therapy, in particular to gene therapy vectors and compositions for treating Parkinson's disease.

### Background of the Disclosure

Parkinson's disease (PD) is a common neurodegenerative disease. PD patients are characterized by the loss of dopaminergic neurons in the substantia nigra region. Dopaminergic neurons are mainly a type of neuron that can produce dopamine, which can transmit nerve impulse signals to the striatum which mainly controls the movements of skeletal muscle. Therefore, the reduction of dopamine synthesis leads to a movement disorder of the body. The main clinical manifestations of the patients are: bradykinesia, myotonia, static tremor, abnormal posture and gait, etc. PD affects about 1% of people over 55 years old in the world. As society ages, the patient population will continue to expand.

The common treatment for Parkinson's disease is oral administration of levodopa, a precursor of dopamine. This drug has a good remission effect in the early stage of treatment, but with the end of the "honeymoon period" of treatment, levodopa can cause a variety of motor complications. In contrast, although dopamine agonist drugs can relieve symptoms to a certain extent, long-term use may lead to adverse reactions such as digestive tract reactions and mental symptoms. For surgical treatment, while it exhibits improvements in symptoms, it can't effect a radical cure. Drug treatment is still needed after surgery, which may also bring related complications such as paralysis of tongue and hands, poor distance perception, and unconsciousness, which also have certain limitations.

Gene therapy has unique advantages for the treatment of Parkinson's disease. On the one hand, a related gene can be introduced into the lesion site through vectors to repair, compensate, and correct the cells in the target area to restore the normal physiological functions of related areas. It is mainly through the introduction of neurotrophic factors (including glial cell-derived neurotrophic factor (GDNF), Neurturin and brain-derived neurotrophic factor (BDNF)) to promote and protect the survival of related neurons. However, a large number of dopaminergic neurons are lost upon the onset of PD in most patients. Therefore, it may be a key to treat this disease by increasing the synthesis of dopamine in the related nerve nuclei of patients. The synthesis of dopamine mainly involves in the following enzymes: tyrosine hydroxylase (TH), aromatic amino acid decarboxylase (AADC), and GTP-cyclohydrolase 1 (GCH1). Tyrosine is catalyzed into levodopa by tyrosine hydroxylase (TH). Levodopa is subsequently converted to dopamine by aromatic amino acid decarboxylase (AADC). TH requires tetrahydrobiopterin as a coenzyme, which is synthesized by GTP-cyclohydrolase 1 (GCH1). Therefore, the expression of TH, AADC, and GCH1 can enable the efficient synthesis of dopamine.

At present, the developing gene therapy strategy for Parkinson's disease targeting dopamine synthetase involves using a lentiviral vector to carry an expression vector containing TH, GCH1, and AADC into the lesion site. By achieving high expression of TH, GCH1, and AADC, this strategy increases the effective concentration of local dopamine in the midbrain striatum site, thus treating Parkinson's disease. This strategy is adopted by a developing drug, AXO-Lenti-PD(OXB-102), which is a Parkinson's disease gene therapy drug targeting dopamine synthetase jointly developed by Oxford Biomedica in the UK and Axovant Gene Therapy in the US. At present, this drug is in the clinical phase I/II research stage. Evidences from animal and clinical trial data demonstrate that the drug can effectively increase the local dopamine in the midbrain striatum and improve the related symptoms of Parkinson's disease. The long-term pharmacodynamic research is still under investigation. However, the three-gene vector packaged by lentivirus exhibits low transduction efficiency and low dopamine synthesis efficiency in nerve cells in vivo, resulting in no significant improvement in the efficacy of pre-clinical model animals and clinical efficacy.

Adeno-associated virus (AAV) vectors belong to the genus *Dependovirus* of Parvoviridae, which is a type of tiny, unenveloped linear single-stranded DNA virus. Because of the high transfection efficiency and high safety, it has become one of the most widely used viral vectors in gene therapy. Due to the limitation in the packaging capacity of AAV vectors, they can only package a DNA fragment within 4.7 kb. At present, there is no clinical trial progress of AAV packaging three genes to express dopamine synthetases for the treatment of Parkinson's disease. The US company Voyager Therapeutics uses AAV2-hAADC as a vector and injects it intracranially into the putamen area of the patient to efficiently express AADC, achieving the treatment of Parkinson's disease by improving the efficiency of converting levodopa into dopamine. However, this therapeutic strategy still relies on the administration of a dopamine precursor drug to the patient, which cannot change the patient's long-term dependence on the dopamine precursor drug, such as Madopar.

There is an urgent need for an optimized gene therapy vector for the treatment of Parkinson's disease.

### Summary of the Invention

In order to address the shortcommings of the existing treatment techniques, the present invention provides an AAV vector encoding three key enzymes for dopamine synthesis, tyrosine hydroxylase (TH), aromatic amino acid decarboxylase (AADC), and GTP-cyclohydrolase 1 (GCH1), wherein any one or more of the TH, GCH1, and AADC is a truncated form of wild type or a functional variant thereof, and the nucleic acid sequences encoding the three enzymes are linked in tandem in a variety of arrangements and linking manners. The present invention overcomes the difficulty of packaging three target genes in an AAV vector by a series of construct optimizations and truncations of the functional regions of the target gene, thereby reducing the size of the fragment constructed by linking the three genes in tandem, and meeting the requirement for packaging the AAV vector. The AAV vector of the present invention can effectively improve the dopamine synthesis efficiency after being delivered into nerve cells in the mouse brain, and achieve an excellent therapeutic effect close to a functional cure in a rat model of Parkinson's disease.

Accordingly, in a first aspect, the present invention provides an adeno-associated virus (AAV) vector comprising nucleic acid sequences encoding tyrosine hydroxylase (TH), GTP-cyclohydrolase 1 (GCH1) and aromatic amino acid decarboxylase (AADC), wherein the nucleic acid sequences encoding the TH, the GCH1 and/or the AADC are in-frame linked by a nucleic acid sequence encoding a linker, wherein any one or more of the TH, the GCH1 and the AADC is a truncated form of wild type or a functional variant thereof and has the catalytic activity required in the dopamine synthesis pathway.

In some embodiments, the TH encoded by the AAV vector of the present invention is a truncated form of wild-type TH or a functional variant thereof and has the activity of catalyzing the synthesis of levodopa from tyrosine. In some embodiments, the TH comprises 330 to 390 amino acid residues. In some embodiments, the TH comprises 330 to 380, 330 to 370, 330 to 360, 330 to 350, 330 to 340, 335 to 340, 335 to 380, 340 to 380, 350 to 380, 360 to 380, or 370 to 380 amino acid residues. In some preferred embodiments, the TH comprises 330 to 340 amino acid residues. In some more preferred embodiments, the TH comprises 335 to 340 amino acid residues, for example, 335, 336, 337, 338, 339, or 340 amino acid residues.

In some embodiments, the TH comprises an amino acid sequence having at least 75%, 80%, 85%, 90%, 95%, 96%, 97%, 98%, 99%, or 100% identity to SEQ ID NO: 11. In some embodiments, the GCH1 comprises an amino acid sequence having at least 75%, 80%, 85%, 90%, 95%, 96%, 97%, 98%, 99%, or 100% identity to SEQ ID NO: 9. In some embodiments, the AADC comprises an amino acid sequence having at least 75%, 80%, 85%, 90%, 95%, 96%, 97%, 98%, 99%, or 100% identity to SEQ ID NO: 7.

In some embodiments, the linker linking the TH, the GCH1, and/or the AADC includes a fusion linker, a 2A peptide linker, and/or an internal ribosome entry site (IRES).

In some embodiments, the fusion linker is a peptide linker. In some embodiments, the peptide linker is selected from (GGS)n, (GGGS)n, or (GGGGS)n, wherein n is an integer from 1 to 5. In some preferred embodiments, the fusion linker is (GGGGS)3.

In some embodiments, the 2A peptide linker comprises a 2A peptide selected from foot-and-mouth disease virus 2A peptide (F2A), porcine teschovirus 2A peptide (P2A), Thosea asign virus 2A peptide (T2A), and/or equine rhinitis virus 2A peptide (E2A). In some embodiments, the N-terminus and/or C-terminus, preferably the N-terminus, of the 2A peptide linker further comprises a GSG amino acid sequence. In some preferred embodiments, the 2A peptide linker comprises an amino acid sequence having at least 75%, 80%, 85%, 90%, 95%, 96%, 97%, 98%, 99%, or 100% identity to SEQ ID NO: 13 or SEQ ID NO: 15.

In some embodiments, the nucleic acid sequences encoding the TH, the GCH1, and the AADC in the AAV vector of the present invention are arranged from upstream to downstream in an order selected from any one of:
(1) TH, GCH1, AADC;
(2) AADC, GCH1, TH;
(3) AADC, TH, GCH1;
(4) GCH1, TH, AADC;
(5) GCH1, AADC, TH; or
(6) TH, AADC, GCH1.

In some embodiments, the nucleic acid sequences encoding the TH, the GCH1, and the AADC in the AAV vector of the present disclosure are linked from upstream to downstream in a manner selected from any one of:
1) TH-F2A-GCH1-P2A-AADC;
2) GCH1-P2A-TH-P2A-AADC;
3) GCH1-P2A-AADC-P2A-TH;
4) AADC-E2A-GCH1-(G4S)3-TH;
5) GCH1-F2A-TH-F2A-AADC;
6) AADC-(G4S)3-TH-T2A-GCH1;
7) AADC-(G4S)4-TH-T2A-GCH1;
8) AADC-P2A-GCH1-P2A-TH;
9) AADC-T2A-GCH1-(G4S)3-TH;
10) GCH1-F2A-TH-P2A-AADC;
11) GCH1-F2A-AADC-F2A-TH;
12) AADC-(G4S)3-GCH1-F2A-TH;
13) GCH1-E2A-TH-T2A-AADC;
14) GCH1-F2A-AADC-P2A-TH;
15) AADC-(G4S)3-GCH1-P2A-TH;
16) AADC-(G4S)5-GCH1-P2A-TH;
17) TH-(G4S)3-GCH1-P2A-AADC;
18) GCH1-T2A-AADC-E2A-TH;
19) AADC-(G4S)3-GCH1-T2A-TH;
20) GCH1-E2A-TH-P2A-AADC;
21) AADC-(G4S)3-GCH1-E2A-TH;
22) GCH1-E2A-AADC-P2A-TH;
23) AADC-(G4S)3-GCH1-(G4S)3-TH;
24) GCH1-T2A-TH-E2A-AADC;
25) GCH1-(G4S)3-TH-T2A-AADC;
26) GCH1-(G4S)2-TH-T2A-AADC;
27) GCH1-P2A-AADC-T2A-TH;
28) TH-P2A-GCH1-P2A-AADC;
29) TH-(G4S)3-GCH1-T2A-AADC;
30) GCH1-(G4S)3-TH-E2A-AADC;
31) GCH1-T2A-TH-(G4S)3-AADC;
32) TH-P2A-GCH1-F2A-AADC;
33) GCH1-F2A-TH-(G4S)3-AADC;
34) TH-F2A-GCH1-F2A-AADC;
35) TH-(G4S)3-GCH1-E2A-AADC;
36) TH-G4S-GCH1-E2A-AADC;
37) GCH1-P2A-TH-(G4S)3-AADC;
38) AADC-F2A-GCH1-P2A-TH;
39) TH-T2A-GCH1-(G4S)3-AADC;
40) GCH1-E2A-TH-(G4S)3-AADC;
41) TH-E2A-GCH1-(G4S)3-AADC;
42) TH-T2A-GCH1-E2A-AADC;
43) AADC-F2A-GCH1-F2A-TH;
44) TH-F2A-GCH1-(G4S)3-AADC;
45) GCH1-(G4S)3-TH-(G4S)3-AADC;
46) TH-(G4S)3-GCH1-F2A-AADC;
47) TH-E2A-GCH1-T2A-AADC;
48) AADC-P2A-TH-(G4S)3-GCH1;
49) TH-(G4S)3-GCH1-(G4S)3-AADC;
50) AADC-F2A-TH-(G4S)3-GCH1;
51) TH-F2A-AADC-F2A-GCH1;
52) TH-(G4S)3-AADC-T2A-GCH1;
53) TH-(G4S)3-AADC-E2A-GCH1;
54) AADC-P2A-GCH1-(G4S)3-TH;
55) TH-F2A-AADC-(G4S)3-GCH1;
56) AADC-F2A-GCH1-(G4S)3-TH;
57) TH-P2A-AADC-F2A-GCH1;
58) TH-P2A-AADC-(G4S)3-GCH1;
59) GCH1-(G4S)3-TH-P2A-AADC;
60) TH-T2A-AADC-(G4S)3-GCH1;
61) TH-T2A-AADC-E2A-GCH1;
62) GCH1-(G4S)3-TH-F2A-AADC;
63) TH-E2A-AADC-(G4S)3-GCH1;
64) AADC-E2A-GCH1-T2A-TH;
65) GCH1-P2A-TH-F2A-AADC;
66) TH-(G4S)3-AADC-(G4S)3-GCH1;
67) TH-E2A-AADC-T2A-GCH1;
68) GCH1-P2A-AADC-F2A-TH;
69) AADC-T2A-TH-(G4S)3-GCH1;
70) AADC-T2A-GCH1-E2A-TH;
71) TH-F2A-AADC-P2A-GCH1;
72) AADC-E2A-TH-(G4S)3-GCH1;
73) AADC-P2A-GCH1-F2A-TH;
74) TH-(G4S)3-AADC-F2A-GCH1;
75) AADC-P2A-TH-F2A-GCH1;
76) AADC-(G4S)3-TH-P2A-GCH1;
77) AADC-T2A-TH-E2A-GCH1;
78) AADC-(G4S)3-TH-E2A-GCH1;
79) AADC-E2A-TH-T2A-GCH1; or
80) AADC-(G4S)3-TH-F2A-GCH1.

In some preferred embodiments, the AAV vector of the present invention comprises a nucleic acid sequence having at least 75%, 80%, 85%, 90%, 95%, 96%, 97%, 98%, 99%, or 100% identity to any one of SEQ ID NOs: 19-21.

In some embodiments, the AAV vector of the present invention further comprises a promoter operably linked to the nucleic acid sequences encoding the TH, the GCH1, and the AADC. In some embodiments, the promoter is selected from a CBH promoter, a chimeric Synapsin I promoter, a CMV promoter, a CAG promoter, a CAGG promoter, or a CASI promoter. In some embodiments, the promoter comprises a sequence having at least 75%, 80%, 85%, 90%, 95%, 96%, 97%, 98%, 99%, or 100% identity to any one of SEQ ID NOs: 1-6. In some preferred embodiments, the promoter comprises the nucleic acid sequence set forth in any one of SEQ ID NOs: 1-3. In some more preferred embodiments, the promoter comprises the nucleic acid sequence set forth in SEQ ID NO: 1.

In some embodiments, the AAV vector of the present invention further comprises one or more elements selected from a KOZAK sequence, a polyadenylation signal of an SV40 virus (SV40 poly A), and an inverted terminal repeat (ITR).

In a second aspect, the present invention provides an AAV virus particle comprising the AAV vector according to the first aspect of the present invention, and a capsid protein. In some embodiments, the capsid protein of the AAV virus particle is derived from an AAV selected from the following serotypes: AAV5, AAV9, AAVPHP.eB, AAVPHP.S or AAVPHP.B. In some embodiments, the capsid protein comprises an amino acid sequence having at least 75%, 80%, 85%, 90%, 95%, 96%, 97%, 98%, 99%, or 100% identity to any one of SEQ ID NOs: 33-37. In some preferred embodiments, the capsid protein comprises an amino acid sequence selected from SEQ ID NO: 34 or 37. In some more preferred embodiments, the capsid protein comprises the amino acid sequence set forth in SEQ ID NO: 34.

In a third aspect, the present invention provides a composition comprising the AAV vector of the first aspect of the present invention and a packaging plasmid encoding a capsid protein, and optionally a helper plasmid.

In some embodiments, the packaging plasmid further comprises a nucleic acid sequence encoding a Rep protein, wherein the Rep protein is derived from an AAV of AAV2 or AAVPHP.B serotype. In some embodiments, the Rep protein comprises an amino acid sequence having at least 75%, 80%, 85%, 90%, 95%, 96%, 97%, 98%, 99%, or 100% identity to SEQ ID NO: 45 or SEQ ID NO: 46. In some preferred embodiments, the Rep protein comprises an amino acid sequence having at least 75%, 80%, 85%, 90%, 95%, 96%, 97%, 98%, 99%, or 100% identity to SEQ ID NO: 46.

In some embodiments, the packaging plasmid in the composition comprises a nucleic acid sequence having at least 75%, 80%, 85%, 90%, 95%, 96%, 97%, 98%, 99%, or 100% identity to any one of SEQ ID NOs: 24-28, 48. In some preferred embodiments, the packaging plasmid comprises a nucleic acid sequence selected from SEQ ID NO: 25 or 28. In some more preferred embodiments, the packaging plasmid comprises the nucleic acid sequence set forth in SEQ ID NO: 25.

In some embodiments, the helper plasmid comprises a nucleic acid sequence having at least 75%, 80%, 85%, 90%, 95%, 96%, 97%, 98%, 99%, or 100% identity to SEQ ID NO: 29.

In some embodiments, the composition further comprises a pharmaceutically acceptable carrier, diluent, or excipient.

In a fourth aspect, the present invention provides a composition comprising the AAV virus particle of the second aspect of the present invention, and optionally a pharmaceutically acceptable carrier, diluent or excipient.

In an embodiment relating to the composition of the third and fourth aspects of the present invention, the pharmaceutically acceptable carrier, diluent or excipient is a buffer. In some embodiments, the buffer comprises a phosphate buffer, sodium chloride, and an F68 surfactant (also known as Poloxamer 188).

In some embodiments, the phosphate buffer is a sodium phosphate buffer or a potassium phosphate buffer. In some preferred embodiments, the phosphate buffer is a sodium phosphate buffer.

In some embodiments, the buffer has a pH of 6.5-8, for example, a pH of 6.6, 6.7, 6.8, 6.9, 7, 7.1, 7.2, 7.3, 7.4, 7.5, 7.6, 7.7, 7.8, or 7.9. In some preferred embodiments, the buffer comprises 5-15 mM phosphate buffer, 130-170 mM sodium chloride, and 0.0005-0.002% F68 surfactant. For example, in the buffer, the concentration of phosphate buffer may be 5, 6, 7, 8, 9, 10, 11, 12, 13, 14, or 15 mM; the concentration of sodium chloride may be 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, or 170 mM; and the content of the F68 surfactant may be 0.0005%, 0.0006%, 0.0007%, 0.0008%, 0.0009%, 0.001%, 0.0011%, 0.0012%, 0.0013%, 0.0014%, 0.0015%, 0.0016%, 0.0017%, 0.0018%, 0.0019%, or 0.002%.

In some embodiments, the buffer comprises 10 mM sodium phosphate buffer, 150 mM sodium chloride, and 0.001% F68 surfactant, with a pH of 7.4.

In a fifth aspect, the present invention provides a cell comprising the AAV vector according to the first aspect, the AAV virus particle according to the second aspect, or the composition according to the third aspect or the fourth aspect of the present invention.

In some embodiments, said cell is a nerve cell, as distinguished by function, the nerve cell is selected from: an inhibitory neuron (preferably, said cell is a medium spiny neuron, an interneuron), an excitatory neuron (preferably, said cell is a dopaminergic neuron), a microglia, an astrocyte, an oligodendrocyte, or a Schwann cell. In some preferred embodiments, as distinguished by brain region, said cell is a midbrain striatal neuron and a glial cell.

In a sixth aspect, the present disclosure provides use of the AAV vector according to the first aspect, the AAV virus particle according to the second aspect, the composition according to the third or fourth aspect, and the cell according to the fifth aspect of the present invention in the manufacture of a medicament for preventing and/or treating a disease caused by dopamine deficiency in a subject. In some embodiments, the disease is Parkinson's disease.

### Description of the Drawings

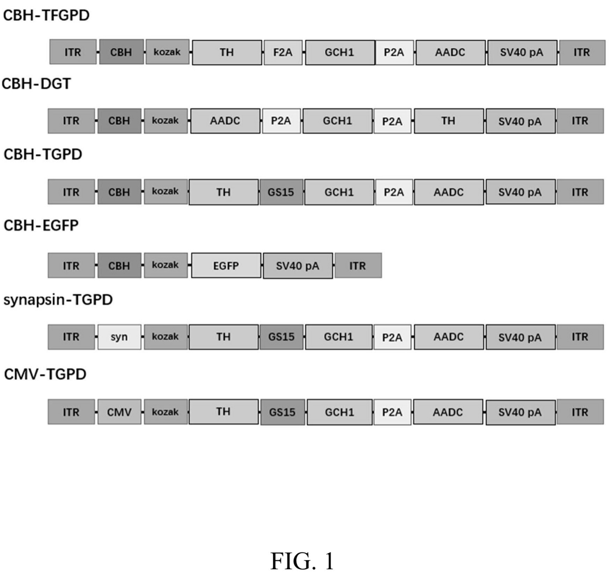

FIG. 1 shows a schematic diagram of the structure of various AAV vectors used in the present invention.

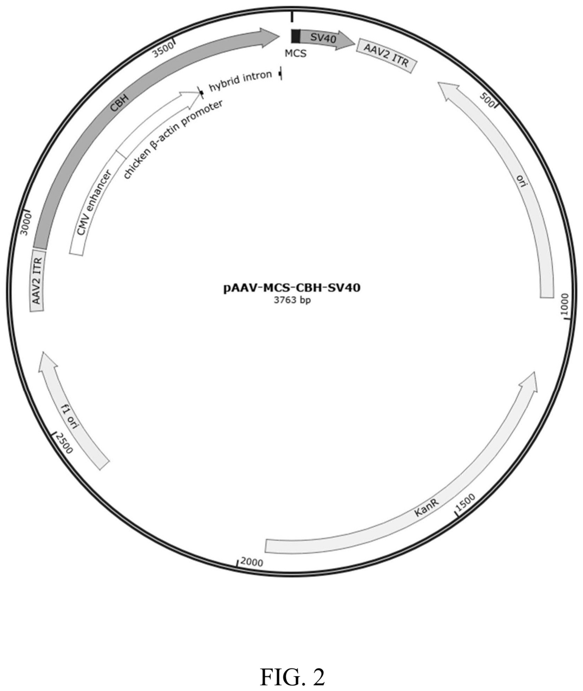

FIG. 2 shows the plasmid profile of pAAV-MCS-CBH-SV40. MCS represents Multiple Cloning Site.

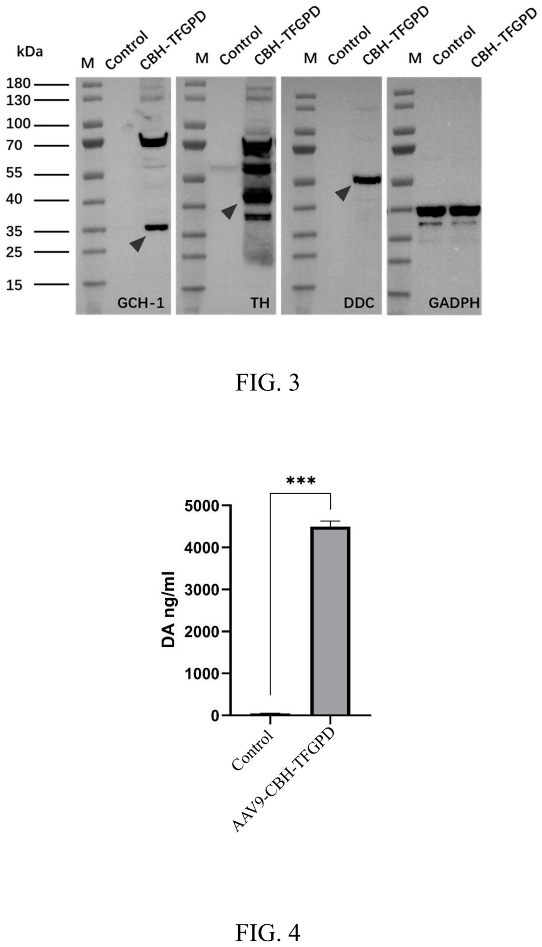

FIG. 3 shows the expressions of proteins of interest in 293T cells after transduction with AAV9-CBH-TFGPD virus determined by Western Blot. AADC represents aromatic amino acid decarboxylase, GCH1 represents GTP-cyclohydrolase 1, TH represents tyrosine hydroxylase, and GAPDH represents glyceraldehyde-3-phosphate dehydrogenase (as an internal reference). The control virus was AAV9-CBH-EGFP.
FIG. 4 shows the concentration of dopamine in the cell supernatant 72 h after infection of 293T cells with AAV-CBH-TFGPD vector in vitro. The error bars represent the standard error (SEM), the control virus was AAV9-CBH-EGFP, and DA represents dopamine. Statistical analysis was performed using one-way ANOVA, ***: p<0.005.

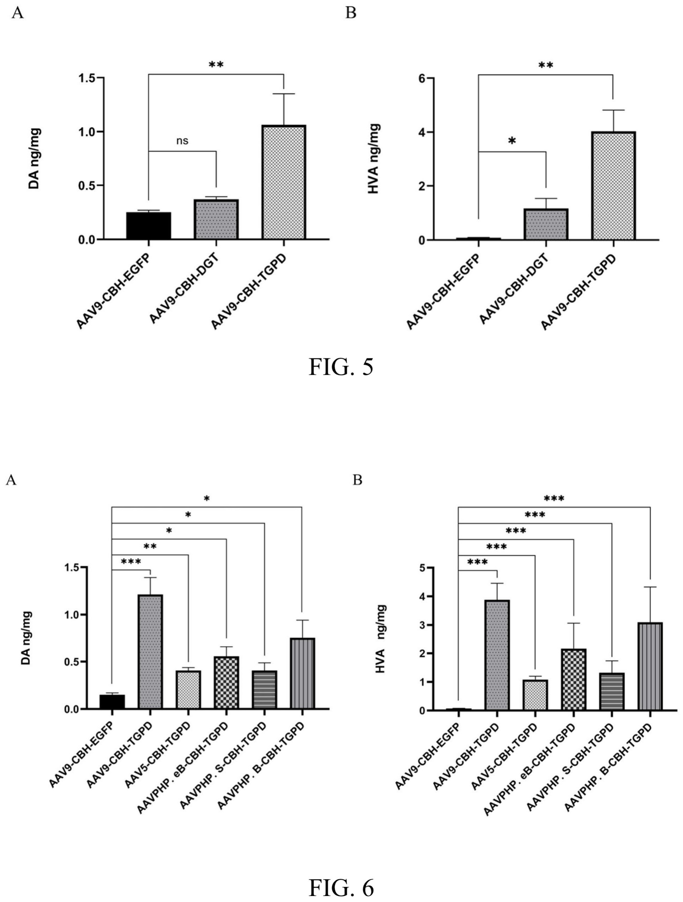

FIG. 5 shows the efficiency of dopamine synthesis mediated by various AAV vectors in mice. The error bars represent the standard error (SEM), the control virus was AAV9-CBH-EGFP, DA represents dopamine, and HVA represents the dopamine metabolite homovanillic acid. Statistical analysis was performed using one-way ANOVA, ns represents no significant difference, *: p<0.05, **: p<0.01, n=3.
FIG. 6 shows the efficiency of dopamine synthesis mediated by AAV vectors with different serotypes in mice. The error bars represent the standard error (SEM), DA represents dopamine, and HVA represents the dopamine metabolite homovanillic acid. Significance analysis was performed using two-tailed t-test, *: p<0.05, **: p<0.01, ***: p<0.001, n=3.

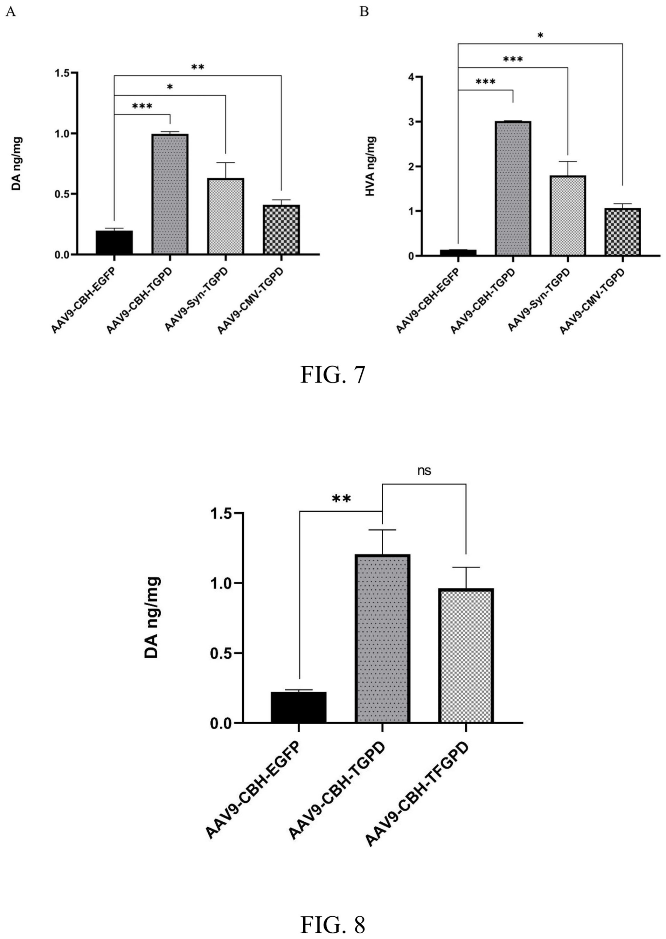

FIG. 7 shows the efficiency of dopamine synthesis mediated by AAV vectors comprising different promoters in mice. The error bars represent the standard error (SEM), DA represents dopamine, and HVA represents the dopamine metabolite homovanillic acid. Significance analysis was performed using two-tailed t-test, *: p<0.05, **: p<0.01, ***: p<0.001, n=3.
FIG. 8 shows the efficiency of dopamine synthesis mediated by AAV vectors comprising different linkers in mice. The error bars represent the standard error (SEM), and DA represents dopamine. Statistical analysis was performed using one-way ANOVA, ns represents no significant difference, **: p<0.01, n=3.

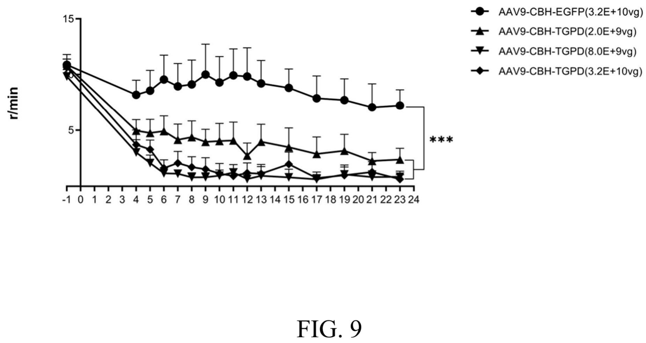

FIG. 9 shows the efficacy of various AAV vectors in a rat model of Parkinson's disease. The line graph represents the APO-induced rotational speed (r/min) for different dosing groups, and the error bars represent the standard error (SEM). Each dosing group with different dosage was compared with the placebo group, and statistical analysis was performed using one-way ANOVA, ***: p<0.001.

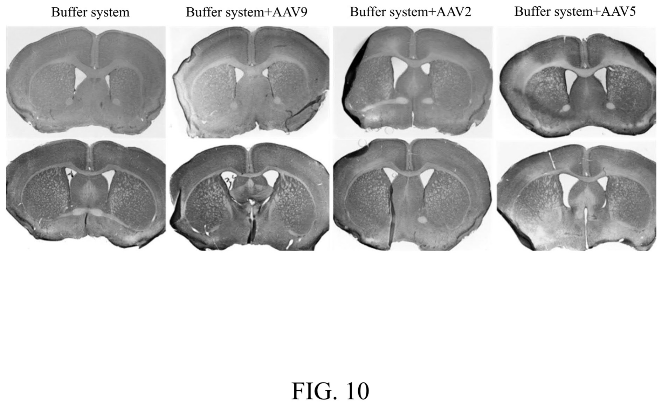

FIG. 10 shows Nissl staining images of the striatum of wild-type C57/B6 mice after injection of the compositions of AAV virus particles and buffer system.

### Detailed Description of the Invention

The present invention provides an AAV vector, an AAV virus particle or a composition useful in highly effective gene therapy for diseases characterized by dopamine deficiency, such as Parkinson's disease. Specifically, the present disclosure provides AAV vectors comprising a series of constructs. Three proteins, i.e., functional tyrosine hydroxylase (TH), GTP-cyclohydrolase 1 (GCH1), and aromatic amino acid decarboxylase (AADC), are linked by linkers, and then cloned into an AAV expression vector. In such embodiments, any one or more of the TH, the GCH1, and the AADC is a truncated form of wild type or a functional variant thereof and has the catalytic activity required in the dopamine synthesis pathway. Preferably, the TH is a truncated form of wild-type TH, and it retains the function of the wild-type TH.

Compared with the prior art, the present invention has at least the following advantages:
1. The AAV vector of the present invention exhibits a significantly enhanced gene transduction efficiency in 293T cells in vitro, and significantly upregulated expression levels of target proteins and increased levels of dopamine and its metabolites;
2. The AAV vectors comprising various constructs of the present invention exhibit a significantly enhanced gene transduction efficiency in nerve cells in vivo, and significantly increased levels of dopamine and its metabolites;
3. The AAV virus particles with various serotypes of the present invention can successfully transduce nerve cells in the mouse brain, and significantly increase the intracellular levels of dopamine and its metabolites;
4. The AAV vectors comprising various promoters of the present invention can successfully transduce nerve cells in the mouse brain, and significantly increase the intracellular levels of dopamine and its metabolites;
5. The AAV vectors comprising various linkers of the present invention can successfully transduce nerve cells in the mouse brain, and significantly increase the intracellular levels of dopamine and its metabolites;
6. The AAV vector of the present invention can significantly improve the motor ability of the rat model of Parkinson's disease. Compared with Prosavin, a lentiviral vector drug used for treating Parkinson's disease (Mimoun Azzouz, et al. The Journal of Neuroscience, 2002, 22(23):10302-10312), the AAV vector of the present invention exhibits a significant improvement in the pharmacodynamic result in rats, and the AAV vector of the present invention shows an obvious dose-effect relationship in the pharmacodynamic model;
7. The present invention breaks through the technical bottleneck. It achieves a remarkable improvement in gene transduction efficiency while carrying three genes on an AAV. Furthermore, the expression levels of target proteins and the levels of dopamine and its metabolites are significantly increased without limitation of promoter, serotype, gene sequence, and linker.

Some embodiments according to the present invention will be described more comprehensively below. However, aspects of the present disclosure may be embodied in different forms and should not be construed as limited to the embodiments set forth herein. Rather, these embodiments are provided in order to render the present invention thorough and complete, and will fully convey the scope of the present disclosure to those skilled in the art. The terms used in the description herein are for the purpose of describing particular embodiments only, and are not intended to be limiting.

Unless otherwise defined, all terms (including technical and scientific terms) used herein have the same meaning as commonly understood by one of ordinary skill in the art to which this invention belongs. It should also be understood that terms such as those defined in commonly used dictionaries should be interpreted as having meanings consistent with their meanings in the context of this application and related fields, and should not be interpreted in an idealized or overly formal sense unless explicitly defined herein.

The various features of the present invention described herein may be used in any combination unless explicitly indicated. Furthermore, it is also contemplated that any feature or combination of features described herein may be excluded or omitted in an embodiment.

Unless explicitly indicated, all specified embodiments, features, and terms are intended to include the recited embodiments, features, or terms as well as their biological equivalents.

All references, articles, publications, patent publications, and patent applications cited herein are incorporated by reference in their entirety for all purposes. However, references to any references, articles, publications, patent publications, and patent applications cited herein shall not and should not be taken as an admission or indication in any form that they constitute a valid prior art or form a part of common knowledge in any country of the world.

### Definition

Unless otherwise indicated, the practice of the present invention will employ conventional techniques of organic chemistry, pharmacology, immunology, molecular biology, microbiology, cell biology, and recombinant DNA, which are within the skill of the art. See, for example, Sambrook, Fritsch, and Maniatis, Molecular Cloning: A Laboratory Manual, 2nd Ed (1989); Current Protocols In Molecular Biology(F.M.Ausubel et al, eds., (1987)); the series Methods in Enzymology(Academic Press,Inc.):PCR 2:A Practical Approach(M.J.MacPherson,B.D.Hames, and G.R.Taylor eds. (1995)),Harlow, and Lane eds., (1988)Antibodies,a Laboratory Manual,and Animal Cell Culture(RI.Freshney, eds. (1987)).

As used herein, the terms "including", "comprising", and "having" are intended to mean that the vector, composition, or method includes the described elements, without excluding other elements.

As used herein, "and/or" refers to and includes any and all possible combinations of one or more of the associated listed items. For example, a composition comprising A and/or B can be interpreted as a composition comprising A, a composition comprising B, or a composition comprising A and B.

All numerical designations, such as pH, temperature, time, concentration, and molecular weight, including ranges, are approximations, which are suitably varied by (+) or (-) in increments of 1.0 or 0.1, or optionally varied by +/- 15%, 10%, 5%, or 2%. It should be understood that all numerical designations are preceded by the term "about". It is also to be understood that the agents described herein are merely exemplary, and equivalents thereof are known in the art. The term "about" as used herein, when referring to measurable values such as amounts or concentrations, refers to including variations within 20%, 10%, 5%, 1%, 0.5%, or 0.1% of a specified amount.

The terms "acceptable", "effective", or "sufficient", as used herein, when used to describe the selection of any component, range, dosage form, etc., mean that the component, range, dosage form, etc., are suitable for the disclosed purposes.

As used herein, the terms "nucleic acid sequence", "nucleotide sequence", and "polynucleotide" are used interchangeably and refer to polymeric forms of nucleotides (ribonucleotides or deoxyribonucleotides) of any length. Accordingly, the term includes, but is not limited to, single-stranded or double-stranded DNA or RNA, genomic DNA, cDNA, DNA-RNA hybrids, or polymers comprising, consisting of, or consisting essentially of purine and pyrimidine bases or other natural, chemically or biochemically modified, non-natural, or derived nucleotide bases.

The terms "protein", "peptide", "polypeptide", and "amino acid sequence" are used interchangeably, and refer in their broadest sense to a polymeric form of two or more amino acid subunits, amino acid analogs, or peptidomimetics. A "protein", a "peptide", a "polypeptide", and an "amino acid sequence" contain at least two amino acids, and there is no limitation on the maximum number of amino acids. As used herein, the term "amino acid" refers to natural and/or non-natural or synthetic amino acids, including D and L optical isomers and amino acid analogs.

"Homology" or "identity" refers to sequence similarity between two polypeptides or between two nucleic acid sequences. Percent identity can be determined by comparing positions in each sequence, which can be aligned for comparison purposes. When a position in the sequence being compared is occupied by the same base or amino acid, the molecules are identical at that position. The degree of identity between sequences depends on the number of shared matching positions. An "unrelated" or "non-homologous" sequence shares less than 40% identity, less than 25% identity with one of the sequences of the present invention. The alignment and sequence identity percentage of the nucleic acid or amino acid sequences provided herein can be determined by introducing the nucleic acid or amino acid sequences into ClustalW (available from https://genome.jp/tools-bin/clustalw/) and using the CluatalW.

The terms "equivalent" or "biological equivalent", when referring to a particular molecule, biological material, or cellular material, are used interchangeably and refer to those having minimal homology while still maintaining a desired structure or function. Non-limiting examples of equivalent polypeptides include a polypeptide having at least about 60%, at least about 65%, at least about 70%, at least about 75%, at least about 80%, at least about 85%, at least about 90%, at least about 95%, or at least about 99% identity to a reference polypeptide (e.g., a wild-type polypeptide); or a polypeptide encoded by a polynucleotide having at least about 70%, at least about 75%, at least about 80%, at least about 85%, at least about 90%, at least about 95%, or at least about 99% identity to a reference polynucleotide (e.g., a wild-type polynucleotide).

As used herein, an amino acid modification can be an amino acid substitution, an amino acid deletion, or an amino acid insertion. The amino acid substitution may be a conservative amino acid substitution or a non-conservative amino acid substitution. A conservative substitution (also referred to as a conservative mutation, conservative replacement, or conservative variation) is an amino acid substitution in a protein that changes a given amino acid to a different amino acid having similar biochemical properties, such as charge, hydrophobicity, or size. As used herein, a "conservative variation" refers to the substitution of an amino acid residue by another biologically similar residue. Examples of conservative variations include the substitution of one hydrophobic residue (such as isoleucine, valine, leucine, or methionine) for another; or one charged or polar residue for another, such as arginine for lysine, glutamic acid for aspartic acid, glutamine for asparagine, and the like. Other exemplary examples of conservative substitutions include the following changes: alanine to serine; asparagine to glutamine or histidine; aspartic acid to glutamic acid; cysteine to serine; glycine to proline; histidine to asparagine or glutamine; lysine to arginine, glutamine or glutamic acid; phenylalanine to tyrosine; serine to threonine; threonine to serine; tryptophan to tyrosine; tyrosine to tryptophan or phenylalanine; and the like.

As used herein, "expression" refers to the process that a nucleic acid sequence is transcribed into an mRNA and/or the process that the transcribed mRNA is subsequently translated into a peptide, polypeptide, amino acid sequence, or protein. If the nucleic acid sequence is from genomic DNA, expression may include splicing the mRNA in a eukaryotic cell.

The term "encoding", when applied to a nucleic acid sequence, refers to a nucleic acid sequence that, in its native state or when operated by methods well known to those skilled in the art, can be transcribed to produce an mRNA and/or translated to produce a polypeptide, and thus is referred to as "encoding" a polypeptide. An antisense strand is the complement of such a nucleic acid, and the coding sequence can be derived therefrom.

The term "promoter" as used herein refers to an expression control sequence that controls the initiation and rate of transcription of a gene or transgene. A promoter may be, for example, constitutive, inducible, repressible, or tissue-specific. A promoter may contain a genetic element to which regulatory proteins and molecules such as RNA polymerases and transcription factors can bind.

Non-limiting examples of promoters include pol I promoter, pol II promoter, pol III promoter, T7 promoter, U6 promoter, H1 promoter, retroviral Rous sarcoma virus LTR promoter, cytomegalovirus (CMV) promoter, SV40 promoter, dihydrofolate reductase promoter, beta-actin promoter, elongation factor 1α short (EFS) promoter, beta glucuronidase (GUSB) promoter, Cytomegalovirus (CMV) immediate early (IE) enhancer and/or promoter, chicken β-actin (CBA) promoter or derivatives thereof such as CAG promoter, CB promoter, (human) elongation factor 1α-subunit (EF1α) promoter, ubiquitin C (UBC) promoter, prion promoter, neuron specific enolase (NSE) promoter, neurofilament light chain (NFL) promoter, neurofilament heavy chain (NFH) promoter, platelet-derived growth factor (PDGF) promoter, platelet-derived growth factor B chain (PDGF-β) promoter, synapsin (Syn) promoter, synapsin 1 (Syn1) promoter, methyl-CpG binding protein 2 (MeCP2) promoter, a Ca2+/calmodulin-dependent protein kinase II (CaMKII) promoter, metabotropic glutamate receptor 2 (mGluR2) promoter, neurofilament light chain (NFL) promoter, neurofilament heavy chain (NFL) promoter,β-globin minigene nβ2 promoter, pre-pro-enkephalin (PPE) promoter, enkephalin (Enk) promoter, excitatory amino acid transporter 2 (EAAT2) promoter, glial fibrillary acidic protein (GFAP) promoter, myelin basic protein (MBP) promoter, or a functional fragment thereof.

It is known in the art that the nucleotide sequences of these promoters can be modified to increase or decrease the efficiency of mRNA transcription. A synthesis-derived promoter can be used for ubiquitous or tissue-specific expression. Furthermore, promoters of viral origin, some of which are described above, such as a CMV, HIV, adenovirus, and AAV promoter, can be used in the methods disclosed herein. In an embodiment, the use of a promoter together with an enhancer can increase transcription efficiency. Non-limiting examples of enhancers include interstitial retinoid binding protein (IRBP) enhancer, RSV enhancer, or CMV enhancer.

As used herein, a "poly A" or "polyA" refers to a string of adenine ribonucleotides or adenosine monophosphates (e.g., a string of RNA in which each base is adenine). This poly A sequence is important for nuclear export, translation, and stability of mRNA. The length of the poly A sequence in various mRNA or RNA sequences of the present invention may vary and may be a poly A of about 250 nucleotides, about 230 nucleotides, about 200 nucleotides, about 180 nucleotides, about 160 nucleotides, about 140 nucleotides, about 120 nucleotides, about 100 nucleotides, or less.

As used herein, a "poly A signal sequence" or "polyadenylation signal sequence" or "polyA signal sequence" refers to an RNA sequence (e.g., AAUAAA) located downstream of the most 3' coding region and recognized by an RNA cleavage complex that cleaves the 3' terminal sequence of a newly transcribed RNA by an RNA polymerase (e.g., Pol II), thereby triggering polyadenylation. Then, polyadenylate polymerase adds and extends the poly A tail by adding adenosine monophosphate units from ATP to the newly cleaved 3' end of the RNA. PolyA signal sequences recognized by RNA cleavage complexes differ across different eukaryotic groups, with most human polyadenylation sites containing AAUAAA sequences, although such sequences are less common in plant and fungal mRNA. All of these sequence motifs recognized by the RNA cleavage complex for RNA cleavage and subsequent polyadenylation are within the scope of the poly A signal sequence.

Exemplary poly A signal sequences include bovine growth hormone polyadenylation signal (bGH poly A), small poly A signal (SPA), human growth hormone polyadenylation signal (hGH poly A), polyadenylation signal of SV40 virus (SV40 poly A), rabbit beta globin poly A sequence (rBG poly A), or variants thereof.

As used herein, the term "vector" refers to a nucleic acid capable of carrying a nucleic acid sequence encoding a protein of interest and capable of promoting expression of the protein of interest when introduced into a cell, for example, by a process of transfection, infection or transformation. It is understood in the art that once introduced into a cell, a vector can replicate as an extrachromosomal (episomal) element or can be integrated into a chromosome of the host cell. Vectors include a plasmid, a cosmid, a phagemid, or an artificial chromosome, and the like. Vectors may include a nucleic acid derived from a retrovirus, adenovirus, adeno-associated virus, herpes virus, baculovirus, or other naturally occurring or modified viruses.

As used herein, a "viral vector" refers to a vector capable of being packaged to recombinantly produce virus particles, which contains a polynucleotide to be delivered into a host cell in vivo, ex vivo, or in vitro. Examples of viral vectors include retroviral vectors, AAV vectors, lentiviral vectors, adenoviral vectors, alphaviral vectors, and the like.

A "gene therapy vector" is defined as any molecule that is able to carry an inserted polynucleotide into a host cell. Examples of gene therapy vectors include liposomes, micelles, biocompatible polymers including natural and synthetic polymers, lipoproteins, polypeptides, polysaccharides, lipopolysaccharides, artificial viral envelopes, metal particles, bacteria, viruses (such as baculoviruses, adenoviruses, and retroviruses), bacteriophages, cosmids, plasmids, fungal vectors, and other recombinant vectors commonly used in the art, which have been described for expression in a variety of eukaryotic and prokaryotic hosts and can be used in gene therapy as well as for protein expression.

A "plasmid" is a DNA molecule that is normally separated from and capable of replicating independently of chromosomal DNA. In many cases, it is cyclic and double-stranded. Plasmids provide a mechanism for horizontal gene transfer within a microbial population and typically provide a selective advantage under a given environmental condition. The plasmid may carry a gene that provides resistance to a naturally occurring antibiotic in a competitive environmental niche, or alternatively, the protein produced may act as a toxin in a similar environment. It is known in the art that although plasmid vectors are typically present as extrachromosomal circular DNA molecules, plasmid vectors can also be designed to stably integrate into the host chromosome in a random or targeted manner, and such integration can be accomplished using circular plasmids or plasmids that have been linearized prior to introduction into the host cell.

A "plasmid" used in genetic engineering is referred to as a "plasmid vector". Many types of plasmids are commercially available for such uses. The gene to be replicated is inserted into a copy of a plasmid containing a gene that allows the cell to be resistant to a specific antibiotic, and a multiple cloning site (MCS or polylinker), which is a short region containing several commonly used restriction sites that allow easy insertion of a DNA fragment at this position. Another major use of plasmids is to produce a large amount of protein. In this case, the researcher cultures a bacterial or eukaryotic cell containing a plasmid carrying a target gene that can be induced to produce a large amount of protein from the inserted gene.

The term "tissue" as used herein refers to the tissue of a living or dead organism or any tissue derived from or designed to mimic a living or dead organism. The tissue may be healthy, diseased, and/or harbor a genetic mutation. A biological tissue may include any single tissue (e.g., a collection of cells that are capable of being interconnected), or a tissue that constitutes a part or region of the body of an organ or organism. The tissue may comprise, consist essentially of, or consist of homogeneous cellular material, or it may be a composite structure, such as a structure found in a region of the body including the thoracic cavity, for example, it may comprise lung tissue, skeletal tissue, and/or muscle tissue. Exemplary tissues include, but are not limited to, those derived from liver, lung, thyroid, skin, pancreas, blood vessel, bladder, kidney, brain, biliary tract, duodenum, abdominal aorta, iliac vein, heart, and intestine, including any combination thereof.

The term "subject" is not limited to a particular species. In some embodiments, the subject includes non-human animals receiving diagnosis or treatment and those animals receiving infections or being animal models, including, but not limited to, simian, murine, rat, canine, or rabbit species, as well as other domestic animals, sporting animals, or pets. In some embodiments, the subject is a human.

As used herein, "treatment" of a disease in a subject refers to (1) prevention of a symptom or disease occurring in a subject that is susceptible to or has not yet exhibited symptoms of the disease; (2) inhibition of the disease or preventing its progression; or (3) amelioration of the symptoms of the disease or induction of resolution of the symptoms. As understood in the art, "treatment" is a method for obtaining beneficial or desired results, including clinical results. For the purposes of the present invention, beneficial or desirable results may include alleviation of one or more diseases or conditions, delay or reduction in the progression thereof, amelioration or mitigation of the state thereof, whether in part or in whole.

The term "effective amount" as used herein means an amount sufficient to achieve the desired effect. In the case of therapeutic or prophylactic applications, the effective amount will depend on the type and severity of the disorder in question and the characteristics of the individual subject, such as general health, age, sex, weight, and tolerance to the drug. In the context of gene therapy, an effective amount is an amount sufficient to cause restoration of partial or all functions of the gene defective in a subject. In some embodiments, an effective amount of the AAV vector is an amount sufficient to result in expression of the gene in a subject. In some embodiments, an effective amount is an amount required to increase the dopamine level in a subject in need thereof.

The effective amount will depend on the nature of the application in question, the individual characteristics and sensitivity of the subject of interest, and the method used. Those skilled in the art will be able to determine an effective amount based on these factors. In some embodiments, the effective amount may comprise one or more administrations of the AAV vector, AAV virus particle, or composition.

As used herein, the term "administration " is intended to mean delivery of a substance to a subject, such as an animal or a human. Administration can be carried out in a single dose, continuously or intermittently throughout the course of treatment. Methods of determining the most effective mode of administration and dosage are known to those skilled in the art. And the most effective mode of administration and dosage will vary depending on factors such as the composition used for treatment, the purpose of the treatment, and the age, health, or sex of the subject being treated. In some embodiments, single or multiple administrations may be performed with dosage and mode selected by a physician or, in the case of pets and other animals, by a veterinarian.

### AAV Vector

"Adeno-associated virus" or "AAV" belongs to the genus *Dependoparvovirus,* the family *Parvoviridae.* AAV is a single-stranded DNA virus that is composed of a viral genome and a capsid. The AAV genome includes the *rep* gene and the *cap* gene, and is flanked by two terminal inverted repeats (ITRs). Among them, the Rep protein encoded by the *rep* gene is related to the replication and packaging of the virus, and the *cap* gene encodes the capsid protein of the virus. An "ITR" or "inverted terminal repeat" may form a T-shaped palindromic structure that is involved in the replication and packaging process of AAV, which is typically necessary to complete the lytic and the latent life cycles of AAVs.

The term "viral capsid" or "capsid" refers to the protein shell of a virus particle. The function of the capsid is to encapsulate, protect, and transport the viral genome, and release it into a host cell. Capsids typically consist of oligomeric structural subunits of proteins (capsid proteins). The viral capsids of AAV consist of a mixture of three viral capsid proteins: VP1, VP2, and VP3.

AAV itself is replication-deficient and can only be latent in host cells in the absence of helper viruses. Therefore, the production of AAV vectors typically requires a helper plasmid to provide key genes involved in AAV replication.

As used herein, "AAV vector" and "recombinant AAV (rAAV) vector" are used interchangeably and mean a vector comprising one or more heterologous nucleic acid sequences and one or more ITRs. When the AAV vector is present in a host cell providing functional *rep* and *cap* gene products, the AAV vector can be replicated and packaged into an infectious virus particle with the help of a helper virus or a helper plasmid.

In some embodiments, the AAV vector of the present invention comprises nucleic acid sequences encoding tyrosine hydroxylase (TH), GTP-cyclohydrolase 1 (GCH1) and aromatic amino acid decarboxylase (AADC), wherein the nucleic acid sequences encoding the TH, the GCH1 and the AADC are in-frame linked by a nucleic acid sequence encoding a linker, wherein any one or more of the TH, the GCH1 and the AADC is a truncated form of wild type or a functional variant thereof and has the catalytic activity required in the dopamine synthesis pathway.

In some embodiments, the TH encoded by the AAV vector of the present invention is a truncated form of wild-type TH or a functional fragment thereof, wherein the truncated form of the TH has the activity of catalyzing the synthesis of levodopa from tyrosine. In some embodiments, the TH comprises 330 to 390 amino acid residues. In some embodiments, the TH comprises 330 to 380, 330 to 370, 330 to 360, 330 to 350, 330 to 340, 335 to 340, 335 to 380, 340 to 380, 350 to 380, 360 to 380, or 370 to 380 amino acid residues. In some preferred embodiments, the TH comprises 330 to 340 amino acid residues. In some more preferred embodiments, the TH comprises 335 to 340 amino acid residues, for example, 335, 336, 337, 338, 339, or 340 amino acid residues.

In some embodiments, the TH comprises an amino acid sequence having at least 75%, 80%, 85%, 90%, 95%, 96%, 97%, 98%, 99%, or 100% identity to SEQ ID NO: 11. In some embodiments, the GCH1 comprises an amino acid sequence having at least 75%, 80%, 85%, 90%, 95%, 96%, 97%, 98%, 99%, or 100% identity to SEQ ID NO: 9. In some embodiments, the AADC comprises an amino acid sequence having at least 75%, 80%, 85%, 90%, 95%, 96%, 97%, 98%, 99%, or 100% identity to SEQ ID NO: 7.

In some embodiments, the linker linking the TH, the GCH1, and/or the AADC includes a fusion linker, a 2A peptide linker, and/or an internal ribosome entry site (IRES).

In some embodiments, the fusion linker is a peptide linker. In some embodiments, the peptide linker is selected from (GGS)n, (GGGS)n or (GGGGS)n, wherein n is an integer from 1 to 5. In some preferred embodiments, the fusion linker is (G4S)3 (SEQ ID NO: 17, GGGGSGGGGSGGGGS).

In some embodiments, the 2A peptide linker comprises a 2A peptide selected from foot-and-mouth disease virus 2A peptide (F2A), porcine teschovirus 2A peptide (P2A), Thosea asign virus 2A peptide (T2A), and/or equine rhinitis virus 2A peptide (E2A).

2A Peptide is a short peptide (about 18-25 amino acids, e.g., ATNFSLLKQAGDVEENPGP) derived from a virus, which can translate a transcript product to produce multiple polypeptide chains. The 2A peptide functions by allowing the ribosome to skip the synthesis of glycine and proline peptide bonds at the C-terminus of the 2A element, ultimately leading to the separation of the terminal of the 2A sequence and the downstream polypeptide chain. After translation of the two proteins linked by the nucleotide sequence encoding the 2A peptide, some additional 2A residues will be added to the C-terminus of the upstream protein, and an additional proline will be added to the N-terminus of the downstream protein.

In some embodiments, the N-terminus and/or C-terminus, preferably the N-terminus, of the 2A peptide linker further comprises a GSG amino acid sequence. In some preferred embodiments, the 2A peptide linker comprises an amino acid sequence having at least 75%, 80%, 85%, 90%, 95%, 96%, 97%, 98%, 99%, or 100% identity to SEQ ID NO: 13 or SEQ ID NO: 15.

In some embodiments, the nucleic acid sequences encoding the TH, the GCH1, and the AADC in the AAV vector of the present invention are arranged from upstream to downstream in an order selected from any one of:
(1) TH, GCH1, AADC;
(2) AADC, GCH1, TH;
(3) AADC, TH, GCH1;
(4) GCH1, TH, AADC;
(5) GCH1, AADC, TH; or
(6) TH, AADC, GCH1.

In some embodiments, the nucleic acid sequences encoding the TH, the GCH1, and the AADC in the AAV vector of the present invention are linked from upstream to downstream in a manner selected from any one of:
1) TH-F2A-GCH1-P2A-AADC;
2) GCH1-P2A-TH-P2A-AADC;
3) GCH1-P2A-AADC-P2A-TH;
4) AADC-E2A-GCH1-(G4S)3-TH;
5) GCH1-F2A-TH-F2A-AADC;
6) AADC-(G4S)3-TH-T2A-GCH1;
7) AADC-(G4S)4-TH-T2A-GCH1;
8) AADC-P2A-GCH1-P2A-TH;
9) AADC-T2A-GCH1-(G4S)3-TH;
10) GCH1-F2A-TH-P2A-AADC;
11) GCH1-F2A-AADC-F2A-TH;
12) AADC-(G4S)3-GCH1-F2A-TH;
13) GCH1-E2A-TH-T2A-AADC;
14) GCH1-F2A-AADC-P2A-TH;
15) AADC-(G4S)3-GCH1-P2A-TH;
16) AADC-(G4S)5-GCH1-P2A-TH;
17) TH-(G4S)3-GCH1-P2A-AADC;
18) GCH1-T2A-AADC-E2A-TH;
19) AADC-(G4S)3-GCH1-T2A-TH;
20) GCH1-E2A-TH-P2A-AADC;
21) AADC-(G4S)3-GCH1-E2A-TH;
22) GCH1-E2A-AADC-P2A-TH;
23) AADC-(G4S)3-GCH1-(G4S)3-TH;
24) GCH1-T2A-TH-E2A-AADC;
25) GCH1-(G4S)3-TH-T2A-AADC;
26) GCH1-(G4S)2-TH-T2A-AADC;
27) GCH1-P2A-AADC-T2A-TH;
28) TH-P2A-GCH1-P2A-AADC;
29) TH-(G4S)3-GCH1-T2A-AADC;
30) GCH1-(G4S)3-TH-E2A-AADC;
31) GCH1-T2A-TH-(G4S)3-AADC;
32) TH-P2A-GCH1-F2A-AADC;
33) GCH1-F2A-TH-(G4S)3-AADC;
34) TH-F2A-GCH1-F2A-AADC;
35) TH-(G4S)3-GCH1-E2A-AADC;
36) TH-G4S-GCH1-E2A-AADC;
37) GCH1-P2A-TH-(G4S)3-AADC;
38) AADC-F2A-GCH1-P2A-TH;
39) TH-T2A-GCH1-(G4S)3-AADC;
40) GCH1-E2A-TH-(G4S)3-AADC;
41) TH-E2A-GCH1-(G4S)3-AADC;
42) TH-T2A-GCH1-E2A-AADC;
43) AADC-F2A-GCH1-F2A-TH;
44) TH-F2A-GCH1-(G4S)3-AADC;
45) GCH1-(G4S)3-TH-(G4S)3-AADC;
46) TH-(G4S)3-GCH1-F2A-AADC;
47) TH-E2A-GCH1-T2A-AADC;
48) AADC-P2A-TH-(G4S)3-GCH1;
49) TH-(G4S)3-GCH1-(G4S)3-AADC;
50) AADC-F2A-TH-(G4S)3-GCH1;
51) TH-F2A-AADC-F2A-GCH1;
52) TH-(G4S)3-AADC-T2A-GCH1;
53) TH-(G4S)3-AADC-E2A-GCH1;
54) AADC-P2A-GCH1-(G4S)3-TH;
55) TH-F2A-AADC-(G4S)3-GCH1;
56) AADC-F2A-GCH1-(G4S)3-TH;
57) TH-P2A-AADC-F2A-GCH1;
58) TH-P2A-AADC-(G4S)3-GCH1;
59) GCH1-(G4S)3-TH-P2A-AADC;
60) TH-T2A-AADC-(G4S)3-GCH1;
61) TH-T2A-AADC-E2A-GCH1;
62) GCH1-(G4S)3-TH-F2A-AADC;
63) TH-E2A-AADC-(G4S)3-GCH1;
64) AADC-E2A-GCH1-T2A-TH;
65) GCH1-P2A-TH-F2A-AADC;
66) TH-(G4S)3-AADC-(G4S)3-GCH1;
67) TH-E2A-AADC-T2A-GCH1;
68) GCH1-P2A-AADC-F2A-TH;
69) AADC-T2A-TH-(G4S)3-GCH1;
70) AADC-T2A-GCH1-E2A-TH;
71) TH-F2A-AADC-P2A-GCH1;
72) AADC-E2A-TH-(G4S)3-GCH1;
73) AADC-P2A-GCH1-F2A-TH;
74) TH-(G4S)3-AADC-F2A-GCH1;
75) AADC-P2A-TH-F2A-GCH1;
76) AADC-(G4S)3-TH-P2A-GCH1;
77) AADC-T2A-TH-E2A-GCH1;
78) AADC-(G4S)3-TH-E2A-GCH1;
79) AADC-E2A-TH-T2A-GCH1; or
80) AADC-(G4S)3-TH-F2A-GCH1.

In some preferred embodiments, the AAV vector of the present invention comprises a nucleic acid sequence having at least 75%, 80%, 85%, 90%, 95%, 96%, 97%, 98%, 99%, or 100% identity to any one of SEQ ID NOs: 19-21.

In some embodiments, the AAV vector of the present invention further comprises a promoter operably linked to the nucleic acid sequences encoding the TH, the GCH1, and the AADC. Promoters suitable for use in the present invention include, but are not limited to: pol I promoter, pol II promoter, pol III promoter, T7 promoter, U6 promoter, H1 promoter, retroviral Rous sarcoma virus LTR promoter, cytomegalovirus (CMV) promoter, SV40 promoter, dihydrofolate reductase promoter, beta-actin promoter, elongation factor 1α short (EFS) promoter, beta glucuronidase (GUSB) promoter, Cytomegalovirus (CMV) immediate early (IE) enhancer and/or promoter, chicken β-actin (CBA) promoter or derivatives thereof such as CAG promoter, CB promoter, (human) elongation factor 1α-subunit (EF1α) promoter, ubiquitin C (UBC) promoter, prion promoter, neuron specific enolase (NSE) promoter, neurofilament light chain (NFL) promoter, neurofilament heavy chain (NFH) promoter, platelet-derived growth factor (PDGF) promoter, platelet-derived growth factor B chain (PDGF-β) promoter, synapsin (Syn) promoter, synapsin I (Syn I) promoter, methyl-CpG binding protein 2 (MeCP2) promoter, a Ca²⁺/calmodulin-dependent protein kinase II (CaMKII) promoter, metabotropic glutamate receptor 2 (mGluR2) promoter, neurofilament light chain (NFL) promoter, neurofilament heavy chain (NFL) promoter, β-globin minigene nβ2 promoter, pre-pro-enkephalin (PPE) promoter, enkephalin (Enk) promoter, excitatory amino acid transporter 2 (EAAT2) promoter, glial fibrillary acidic protein (GFAP) promoter, myelin basic protein (MBP) promoter, or a functional fragment thereof.

In some embodiments, the promoter is selected from a CBH promoter, a chimeric Synapsin I promoter, a CMV promoter, a CAG promoter, a CAGG promoter, or a CASI promoter. In some embodiments, the promoter comprises a nucleic acid sequence having at least 75%, 80%, 85%, 90%, 95%, 96%, 97%, 98%, 99%, or 100% identity to any one of SEQ ID NOs: 1-6. In some preferred embodiments, the promoter comprises a nucleic acid sequence having at least 75%, 80%, 85%, 90%, 95%, 96%, 97%, 98%, 99%, or 100% identity to any one of SEQ ID NOs: 1-3. In some more preferred embodiments, the promoter comprises a nucleic acid sequence having at least 75%, 80%, 85%, 90%, 95%, 96%, 97%, 98%, 99%, or 100% identity to SEQ ID NO: 1. In some most preferred embodiments, the promoter comprises the nucleic acid sequence set forth in SEQ ID NO: 1.

In some embodiments, the AAV vector of the present invention further comprises a poly A signal sequence downstream of the nucleic acid sequences encoding the TH, the GCH1, and the AADC. Exemplary poly A signal sequences include bovine growth hormone polyadenylation signal (bGH poly A), small poly A signal (SPA), human growth hormone polyadenylation signal (hGH poly A), polyadenylation signal of SV40 virus (SV40 poly A), rabbit beta globin poly A sequence (rBG poly A), or variants thereof.

In some embodiments, the AAV vector of the present invention comprises, from 5' to 3', a 5' ITR of AAV, a promoter, a KOZAK sequence, nucleic acid sequences encoding three proteins of the TH, the GCH1, and the AADC (arranged in different orders and linking manners), a poly A signal sequence, and a 3' ITR of AAV.

In some specific embodiments, the AAV vector of the present invention comprises, from 5' to 3':
(1) 5'ITR-CBH promoter-KOZAK-TH-F2A-GCH1-P2A-AADC-SV40 polyA-3'ITR;
(2) 5'ITR-CBH promoter-KOZAK-AADC-P2A-GCH1-P2A-TH-SV40 polyA-3'ITR;
(3) 5'ITR-CBH promoter-KOZAK-TH-GS15-GCH1-P2A-AADC-SV40 polyA-3'ITR;
(4) 5'ITR-chimeric Synapsin I promoter-KOZAK-TH-GS15-GCH1-P2A-AADC-SV40 polyA-3'ITR; or
(5) 5'ITR-CMV promoter-KOZAK-TH-GS15-GCH1-P2A-AADC-SV40 polyA-3'ITR; wherein GS15 is a (G4S)3 linker comprising the amino acid sequence set forth in SEQ ID NO: 17 (GGGGSGGGGSGGGGS).

In some embodiments, the KOZAK sequence comprises a GCCACC nucleic acid sequence. In some embodiments, SV40 polyA comprises a nucleic acid sequence set forth in SEQ ID NO: 47.

### AAV Virus Particle

The present invention provides an AAV virus particle comprising the AAV vector of the present invention, and a capsid protein.

At present, there are more than ten common serotypes and hundreds of variants of AAVs. Their main difference lies in the difference in the *cap* gene encoding the capsid protein. The serotypes of AAV are primarily determined by the structure of the capsid protein and include, but are not limited to: AAV1, AAV2, AAV3, AAV4, AAV5, AAV6, AAV7, AAV8, AAV9, AAV10, AAV11, AAV12, AAV13, AAVrh10, AAV-DJ, AAV-DJ/B, AAV PHP.B, AAV PHP.eB, AAV PHP.S, and AAVrh74.

In some embodiments, the capsid protein is derived from an AAV selected from the following serotypes: AAV1, AAV2, AAV5, AAV6, AAV8, AAV9, AAVrh10, AAV-DJ, AAV PHP.B, AAV PHP.eB, or AAV PHP.S. In some embodiments, the capsid protein is derived from an AAV selected from the following serotypes: AAV5, AAV9, AAVPHP.eB, AAVPHP.S or AAVPHP.B.

In some embodiments, the capsid protein of the AAV virus particle of the present invention comprises an amino acid sequence having at least 75%, 80%, 85%, 90%, 95%, 96%, 97%, 98%, 99%, or 100% identity to any one of SEQ ID NOs: 33-37. In some preferred embodiments, the capsid protein of the AAV virus particle of the present invention comprises an amino acid sequence having at least 75%, 80%, 85%, 90%, 95%, 96%, 97%, 98%, 99%, or 100% identity to SEQ ID NO: 34 or 37. In some more preferred embodiments, the capsid protein of the AAV virus particle of the present invention comprises an amino acid sequence having at least 75%, 80%, 85%, 90%, 95%, 96%, 97%, 98%, 99%, or 100% identity to SEQ ID NO: 34. In some of the most preferred embodiments, the capsid protein of the AAV virus particle of the present invention comprises the amino acid sequence set forth in SEQ ID NO: 34.

In some preferred embodiments, the AAV virus particle of the present invention comprises an AAV vector and a capsid protein, wherein the AAV vector comprises, from 5' to 3': a 5'ITR-CBH promoter-KOZAK-TH-F2A-GCH1-P2A-AADC-SV40 polyA-3'ITR; wherein the capsid protein comprises an amino acid sequence having at least 75%, 80%, 85%, 90%, 95%, 96%, 97%, 98%, 99%, or 100% identity to SEQ ID NO: 34.

In some preferred embodiments, the AAV virus particle of the present invention comprises an AAV vector and a capsid protein, wherein the AAV vector comprises, from 5' to 3': a 5'ITR-CBH promoter-KOZAK-AADC-P2A-GCH1-P2A-TH-SV40 polyA-3'ITR; wherein the capsid protein comprises an amino acid sequence having at least 75%, 80%, 85%, 90%, 95%, 96%, 97%, 98%, 99%, or 100% identity to SEQ ID NO: 34.

In some preferred embodiments, the AAV virus particle of the present invention comprises an AAV vector and a capsid protein, wherein the AAV vector comprises, from 5' to 3': a 5'ITR-CBH promoter-KOZAK-TH-GS15-GCH1-P2A-AADC-SV40 polyA-3'ITR; wherein the capsid protein comprises an amino acid sequence having at least 75%, 80%, 85%, 90%, 95%, 96%, 97%, 98%, 99%, or 100% identity to SEQ ID NO: 34.

In some preferred embodiments, the AAV virus particle of the present invention comprises an AAV vector and a capsid protein, wherein the AAV vector comprises, from 5' to 3': a 5'ITR-chimeric Synapsin I promoter-KOZAK-TH-GS15-GCH1-P2A-AADC-SV40 polyA-3'ITR; wherein the capsid protein comprises an amino acid sequence having at least 75%, 80%, 85%, 90%, 95%, 96%, 97%, 98%, 99%, or 100% identity to SEQ ID NO: 34.

In some preferred embodiments, the AAV virus particle of the present invention comprises an AAV vector and a capsid protein, wherein the AAV vector comprises, from 5' to 3': a 5'ITR-CMV promoter-KOZAK-TH-GS15-GCH1-P2A-AADC-SV40 polyA-3'ITR; wherein the capsid protein comprises an amino acid sequence having at least 75%, 80%, 85%, 90%, 95%, 96%, 97%, 98%, 99%, or 100% identity to SEQ ID NO: 34.

In some preferred embodiments, the AAV virus particle of the present invention comprises an AAV vector and a capsid protein, wherein the AAV vector comprises the nucleotide sequence set forth in any one of SEQ ID NOs: 19-21, and the capsid protein comprises the amino acid sequence set forth in SEQ ID NO: 34.

### Composition

In one aspect, the present invention provides a composition comprising the AAV vector of the present invention and a packaging plasmid encoding a capsid protein, and optionally a helper plasmid.

In some embodiments, the packaging plasmid further comprises a nucleic acid sequence encoding a Rep protein, wherein the Rep protein is derived from an AAV of AAV2 or AAVPHP.B serotype. In some embodiments, the Rep protein comprises an amino acid sequence having at least 75%, 80%, 85%, 90%, 95%, 96%, 97%, 98%, 99%, or 100% identity to SEQ ID NO: 45 or SEQ ID NO: 46.

In some embodiments, the packaging plasmid in the composition comprises a nucleic acid sequence having at least 75%, 80%, 85%, 90%, 95%, 96%, 97%, 98%, 99%, or 100% identity to any one of SEQ ID NOs: 24-28 and 48. In some preferred embodiments, the packaging plasmid comprises a nucleic acid sequence having at least 75%, 80%, 85%, 90%, 95%, 96%, 97%, 98%, 99%, or 100% identity to SEQ ID NO: 25 or 28. In some more preferred embodiments, the packaging plasmid comprises a nucleic acid sequence having at least 75%, 80%, 85%, 90%, 95%, 96%, 97%, 98%, 99%, or 100% identity to SEQ ID NO: 25. In some of the most preferred embodiments, the packaging plasmid comprises the nucleic acid sequence set forth in SEQ ID NO: 25.

In some embodiments, the helper plasmid in the composition comprises a nucleic acid sequence having at least 75%, 80%, 85%, 90%, 95%, 96%, 97%, 98%, 99%, or 100% identity to SEQ ID NO: 29. In some embodiments, the helper plasmid in the composition comprises the nucleic acid sequence set forth in SEQ ID NO: 29.

In another aspect, the present disclosure provides a composition comprising an AAV virus particle of the present invention, wherein the AAV virus particle comprises an AAV vector of the present invention and a capsid protein.

In some embodiments, the composition of the present invention further comprises a pharmaceutically acceptable carrier, diluent, or excipient.

The term "pharmaceutically acceptable" as used herein means that a carrier, excipient, or diluent is suitable for use in contact with tissues of humans and animals without undue toxicity, irritation, allergic response, or other problems or complications within the scope of reasonable medical judgment, and commensurate with a reasonable benefit/risk ratio.

Exemplary carriers used in the compositions of the present invention include saline, buffered saline, dextrose and water. Exemplary excipients used in the compositions of the present invention include a filler, a binder, a disintegrant, a coating agent, an adsorbent, an anti-adhesion agent, a glidant, a preservative, an antioxidant, a flavoring agent, a coloring agent, a sweetening agent, a solvent, a co-solvent, a buffer, a chelating agent, a thickening agent, a surfactant, a diluent, a wetting agent, a carrier, a diluent, a preservative, an emulsifier, a stabilizer, and a tonicity modifier. The selection of suitable excipients to prepare the compositions of the present invention is known to those skilled in the art. In general, the selection of suitable excipients depends, inter alia, on the active agent used, the disease to be treated, and the desired dosage form of the composition.

In some embodiments with respect to the composition, the pharmaceutically acceptable carrier, diluent, or excipient is a buffer. In some embodiments, the buffer comprises a phosphate buffer, sodium chloride, and a surfactant.

In some embodiments, the surfactant is selected from one or more of polyoxyethylene polyoxypropylene ether block copolymer (Poloxamer 188, F68), polysorbate (Tween), polyethylene glycol (PEG), polyethylene glycol p-isooctylphenyl ether (Triton), fatty acid sorbitan (Span), sucrose fatty acid ester (SE), polyoxyethylene fatty alcohol ether (Brij), and polyoxyethylene fatty acid ester (Myeij).

In some embodiments, the surfactant is selected from F68, Tween, or a combination thereof.

In some embodiments, Tween includes Tween 20.

In some embodiments, the phosphate buffer is a sodium phosphate buffer or a potassium phosphate buffer. In some preferred embodiments, the phosphate buffer is a sodium phosphate buffer.

In some embodiments, the buffer has a pH of 6.5-8, for example, a pH of 6.6, 6.7, 6.8, 6.9, 7, 7.1, 7.2, 7.3, 7.4, 7.5, 7.6, 7.7, 7.8, or 7.9. In some preferred embodiments, the buffer comprises 5-15 mM phosphate buffer, 130-170 mM sodium chloride, and 0.0005-0.002% F68. For example, in the buffer, the concentration of phosphate buffer may be 5, 6, 7, 8, 9, 10, 11, 12, 13, 14, or 15 mM; the concentration of sodium chloride may be 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, or 170 mM; and the content of the F68 may be 0.0005%, 0.0006%, 0.0007%, 0.0008%, 0.0009%, 0.001%, 0.0011%, 0.0012%, 0.0013%, 0.0014%, 0.0015%, 0.0016%, 0.0017%, 0.0018%, 0.0019%, or 0.002%.

In some embodiments, the buffer comprises 10 mM sodium phosphate buffer, 150 mM sodium chloride, and 0.001% F68, with a pH of 7.4.

### Examples

In order to make the objects, technical solutions, and advantages of the present invention clearer, the present invention will be further described below with reference to specific examples and the drawings, and the advantages and features of the present invention will become clearer with the description. It should be understood that these examples are merely used to illustrate the present invention and are not intended to limit the scope of the present invention. Experimental procedures for which specific conditions are not indicated in the following examples are performed under conditions conventional in the art, for example, those described in Sambrook and Russeii et al., Molecular Cloning: A Laboratory Manual (3rd Edition) (2001), CSHL Press, or as recommended by the manufacturer. The experimental materials and reagents used in the following examples are commercially available unless otherwise indicated.

### Example 1. Design and construction of AAV vectors

The core sequences used to construct the AAV vectors of the present invention are shown in Table 1 below.

**Table 1. The sequences of promoters, proteins, and partial linkers used in the present invention.**

| | | |
|---|---|---|
| SEQ ID NO: 1 | CBH promoter | CGTTACATAACTTACGGTAAATGGCCCGCCTGGCTGACCGCCCAAC GACCCCCGCCCATTGACGTCAATAATGACGTATGTTCCCATAGTAA CGCCAATAGGGACTTTCCATTGACGTCAATGGGTGGAGTATTTACG GTAAACTGCCCACTTGGCAGTACATCAAGTGTATCATATGCCAAGT ACGCCCCCTATTGACGTCAATGACGGTAAATGGCCCGCCTGGCATT ATGCCCAGTACATGACCTTATGGGACTTTCCTACTTGGCAGTACAT CTACGTATTAGTCATCGCTATTACCATGGTCGAGGTGAGCCCCACG TTCTGCTTCACTCTCCCCATCTCCCCCCCCTCCCCACCCCCAATTTT GTATTTATTTATTTTTTAATTATTTTGTGCAGCGATGGGGGCGGGG GGGGGGGGGGGGCGCGCGCCAGGCGGGGCGGGGCGGGGCGAGGG GCGGGGCGGGGCGAGGCGGAGAGGTGCGGCGGCAGCCAATCAGA GCGGCGCGCTCCGAAAGTTTCCTTTTATGGCGAGGCGGCGGCGGC GGCGGCCCTATAAAAAGCGAAGCGCGCGGCGGGCGGGAGTCGCT GCGACGCTGCCTTCGCCCCGTGCCCCGCTCCGCCGCCGCCTCGCGC CGCCCGCCCCGGCTCTGACTGACCGCGTTACTCCCACAGGTGAGCG GGCGGGACGGCCCTTCTCCTCCGGGCTGTAATTAGCTGAGCAAGA GGTAAGGGTTTAAGGGATGGTTGGTTGGTGGGGTATTAATGTTTAA TTACCTGGAGCACCTGCCTGAAATCACTTTTTTTCAGGT |
| SEQ ID NO: 2 | Synapsin I promoter | CGTTACATAACTTACGGTAAATGGCCCGCCTGGCTGACCGCCCAAC GACCCCCGCCCATTGACGTCAATAATGACGTATGTTCCCATAGTAA CGCCAATAGGGACTTTCCATTGACGTCAATGGGTGGAGTATTTACG GTAAACTGCCCACTTGGCAGTACATCAAGTGTATCATATGCCAAGT ACGCCCCCTATTGACGTCAATGACGGTAAATGGCCCGCCTGGCATT ATGCCCAGTACATGACCTTATGGGACTTTCCTACTTGGCAGTACAT CTACGTATTAGTCATCGCTATTACCATGGCTGCAGAGGGCCCTGCG TATGAGTGCAAGTGGGTTTTAGGACCAGGATGAGGCGGGGTGGGG GTGCCTACCTGACGACCGACCCCGACCCACTGGACAAGCACCCAA CCCCCATTCCCCAAATTGCGCATCCCCTATCAGAGAGGGGGAGGG GAAACAGGATGCGGCGAGGCGCGTGCGCACTGCCAGCTTCAGCAC CGCGGACAGTGCCTTCGCCCCCGCCTGGCGGCGCGCGCCACCGCC GCCTCAGCACTGAAGGCGCGCTGACGTCACTCGCCGGTCCCCCGC AAACTCCCCTTCCCGGCCACCTTGGTCGCGTCCGCGCCGCCGCCGG CCCAGCCGGACCGCACCACGCGAGGCGCGAGATAGGGGGGCACG GGCGCGACCATCTGCGCTGCGGCG |
| SEQ ID NO: 3 | CMV promoter | GACATTGATTATTGACTAGTTATTAATAGTAATCAATTACGGGGTC ATTAGTTCATAGCCCATATATGGAGTTCCGCGTTACATAACTTACG GTAAATGGCCCGCCTGGCTGACCGCCCAACGACCCCCGCCCATTG ACGTCAATAATGACGTATGTTCCCATAGTAACGCCAATAGGGACTT TCCATTGACGTCAATGGGTGGAGTATTTACGGTAAACTGCCCACTT GGCAGTACATCAAGTGTATCATATGCCAAGTACGCCCCCTATTGAC GTCAATGACGGTAAATGGCCCGCCTGGCATTATGCCCAGTACATG ACCTTATGGGACTTTCCTACTTGGCAGTACATCTACGTATTAGTCA TCGCTATTACCATGGTGATGCGGTTTTGGCAGTACATCAATGGGCG TGGATAGCGGTTTGACTCACGGGGATTTCCAAGTCTCCACCCCATT GACGTCAATGGGAGTTTGTTTTGGCACCAAAATCAACGGGACTTTC CAAAATGTCGTAACAACTCCGCCCCATTGACGCAAATGGGCGGTA GGCGTGTACGGTGGGAGGTCTATATAAGCAG |
| SEQ ID NO: 4 | CAG promoter | CCATTGACGTCAATAATGACGTATGTTCCCATAGTAACGCCAATAG GGACTTTCCATTGACGTCAATGGGTGGAGTATTTACGGTAAACTGC CCACTTGGCAGTACATCAAGTGTATCATATGCCAAGTACGCCCCCT ATTGACGTCAATGACGGTAAATGGCCCGCCTGGCATTATGCCCAGT ACATGACCTTATGGGACTTTCCTACTTGGCAGTACATCTACGTATT AGTCATCGCTATTACCATGGTCGAGGTGAGCCCCACGTTCTGCTTC ACTCTCCCCATCTCCCCCCCCTCCCCACCCCCAATTTTGTATTTATT TATTTTTTAATTATTTTGTGCAGCGATGGGGGCGGGGGGGGGGGG GGGGCGCGCGCCAGGCGGGGCGGGGCGGGGCGAGGGGCGGGGCG GGGCGAGGCGGAGAGGTGCGGCGGCAGCCAATCAGAGCGGCGCG CTCCGAAAGTTTCCTTTTATGGCGAGGCGGCGGCGGCGGCGGCCCT ATAAAAAGCGAAGCGCGCGGCGGGCGGGAGTCGCTGCGCGCTGCC TTCGCCCCGTGCCCCGCTCCGCCGCCGCCTCGCGCCGCCCGCCCCG GCTCTGACTGACCGCGTTACTCCCACAGGTGAGCGGGCGGGACGG CCCTTCTCCTCCGGGCTGTAATTAGCGCTTGGTTTAATGACGGCTT GTTTCTTTTCTGTGGCTGCGTGAAAGCCTTGAGGGGCTCCGGGAGG GCCCTTTGTGCGGGGGGAGCGGCTCGGGGCTGTCCGCGGGGGGAC GGCTGCCTTCGGGGGGGACGGGGCAGGGCGGGGTTCGGCTTCTGG CGTGTGACCGGCGGCTCTAGAGCCTCTGCTAACCATGTTCATGCCT TCTTCTTTTTCCTACAGCTCCTGGGCAACGT |
| SEQ ID NO: 5 | CAGG promoter | GTTACATAACTTACGGTAAATGGCCCGCCTGGCTGACCGCCCAAC GACCCCCGCCCATTGACGTCAATAATGACGTATGTTCCCATAGTAA CGCCAATAGGGACTTTCCATTGACGTCAATGGGTGGAGTATTTACG GTAAACTGCCCACTTGGCAGTACATCAAGTGTATCATATGCCAAGT ACGCCCCCTATTGACGTCAATGACGGTAAATGGCCCGCCTGGCATT ATGCCCAGTACATGACCTTATGGGACTTTCCTACTTGGCAGTACAT CTACGTATTAGTCATCGCTATTACCATGGTCGAGGTGAGCCCCACG TTCTGCTTCACTCTCCCCATCTCCCCCCCCTCCCCACCCCCAATTTT GTATTTATTTATTTTTTAATTATTTTGTGCAGCGATGGGGGCGGGG GGGGGGGGGGGGCGCGCGCCAGGCGGGGCGGGGCGGGGCGAGGG GCGGGGCGGGGCGAGGCGGAGAGGTGCGGCGGCAGCCAATCAGA GCGGCGCGCTCCGAAAGTTTCCTTTTATGGCGAGGCGGCGGCGGC GGCGGCCCTATAAAAAGCGAAGCGCGCGGCGGGCGGGGAGTCGC TGCGACGCTGCCTTCGCCCCGTGCCCCGCTCCGCCGCCGCCTCGCG CCGCCCGCCCCGGCTCTGACTGACCGCGTTACTCCCACAGGTGAGC GGGCGGGACGGCCCTTCTCCTCCGGGCTGTAATTAGCGCTTGGTTT AATGACGGCTTGTTTCTTTTCTGTGGCTGCGTGAAAGCCTTGAGGG GCTCCGGGAGGGCCCTTTGTGCGGGGGGAGCGGCTCGGGGGGTGC GTGCGTGTGTGTGTGCGTGGGGAGCGCCGCGTGCGGCTCCGCGCT GCCCGGCGGCTGTGAGCGCTGCGGGCGCGGCGCGGGGCTTTGTGC GCTCCGCAGTGTGCGCGAGGGGAGCGCGGCCGGGGGCGGTGCCCC GCGGTGCGGGGGGGGCTGCGAGGGGAACAAAGGCTGCGTGCGGG GTGTGTGCGTGGGGGGGTGAGCAGGGGGTGTGGGCGCGTCGGTCG GGCTGCAACCCCCCCTGCACCCCCCTCCCCGAGTTGCTGAGCACGG CCCGGCTTCGGGTGCGGGGCTCCGTACGGGGCGTGGCGCGGGGCT CGCCGTGCCGGGCGGGGGGTGGCGGCAGGTGGGGGTGCCGGGCG GGGCGGGGCCGCCTCGGGCCGGGGAGGGCTCGGGGGAGGGGCGC GGCGGCCCCCGGAGCGCCGGCGGCTGTCGAGGCGCGGCGAGCCGC AGCCATTGCCTTTTATGGTAATCGTGCGAGAGGGCGCAGGGACTTC CTTTGTCCCAAATCTGTGCGGAGCCGAAATCTGGGAGGCGCCGCC GCACCCCCTCTAGCGGGCGCGGGGCGAAGCGGTGCGGCGCCGGCA GGAAGGAAATGGGCGGGGAGGGCCTTCGTGCGTCGCCGCGCCGCC GTCCCCTTCTCCCTCTCCAGCCTCGGGGCTGTCCGCGGGGGGACGG CTGCCTTCGGGGGGGACGGGGCAGGGCGGGGTTCGGCTTCTGGCG TGTGACCGGCGGCTCTAGAGCCTCTGCTAACCATGTTCATGCCTTC TTCTTTTTCCTACAG |
| SEQ ID NO: 6 | CASI promoter | GTTACATAACTTACGGTAAATGGCCCGCCTGGCTGACCGCCCAAC GACCCCCGCCCATTGACGTCAATAATGACGTATGTTCCCATAGTAA CGCCAATAGGGACTTTCCATTGACGTCAATGGGTGGAGTATTTACG GTAAACTGCCCACTTGGCAGTACATCAAGTGTATCATATGCCAAGT ACGCCCCCTATTGACGTCAATGACGGTAAATGGCCCGCCTGGCATT ATGCCCAGTACATGACCTTATGGGACTTTCCTACTTGGCAGTACAT CTACGTATTAGTCATCGCTATTACCATGGTCGAGGTGAGCCCCACG TTCTGCTTCACTCTCCCCATCTCCCCCCCCTCCCCACCCCCAATTTT GTATTTATTTATTTTTTAATTATTTTGTGCAGCGATGGGGGCGGGGG GGGGGGGGGGGCGCGCGCCAGGCGGGGCGGGGCGGGGCGAGGG GCGGGGCGGGGCGAGGCGGAGAGGTGCGGCGGCAGCCAATCAGA GCGGCGCGCTCCGAAAGTTTCCTTTTATGGCGAGGCGGCGGCGGC GGCGGCCCTATAAAAAGCGAAGCGCGCGGCGGGCGGGAGTCGCT GCGCGCTGCCTTCGCCCCGTGCCCCGCTCCGCCGCCGCCTCGCGCC GCCCGCCCCGGCTCTGACTGACCGCGTTACTAAAACAGGTAAGTC CGGCCTCCGCGCCGGGTTTTGGCGCCTCCCGCGGGCGCCCCCCTCC TCACGGCGAGCGCTGCCACGTCAGACGAAGGGCGCAGCGAGCGTC CTGATCCTTCCGCCCGGACGCTCAGGACAGCGGCCCGCTGCTCATA AGACTCGGCCTTAGAACCCCAGTATCAGCAGAAGGACATTTTAGG ACGGGACTTGGGTGACTCTAGGGCACTGGTTTTCTTTCCAGAGAGC GGAACAGGCGAGGAAAAGTAGTCCCTTCTCGGCGATTCTGCGGAG GGATCTCCGTGGGGCGGTGAACGCCGATGATGCCTCTACTAACCA TGTTCATGTTTTCTTTTTTTTTCTACAGGTCCTGGGTGACGAACAGG CTAGC |
| SEQ ID NO: 7 | AADC amino acid sequence | MNASEFRRRGKEMVDYMANYMEGIEGRQVYPDVEPGYLRPLIPAAA PQEPDTFEDIINDVEKIIMPGVTHWHSPYFFAYFPTASSYPAMLADML CGAIGCIGFSWAASPACTELETVMMDWLGKMLELPKAFLNEKAGEG GGVIQGSASEATLVALLAARTKVIHRLQAASPELTQAAIMEKLVAYSS DQAHSSVERAGLIGGVKLKAIPSDGNFAMRASALQEALERDKAAGLI PFFMVATLGTTTCCSFDNLLEVGPICNKEDIWLHVDAAYAGSAFICPE FRHLLNGVEFADSFNFNPHKWLLVNFDCSAMWVKKRTDLTGAFRLD PTYLKHSHQDSGLITDYRHWQIPLGRRFRSLKMWFVFRMYGVKGLQ AYIRKHVQLSHEFESLVRQDPRFEICVEVILGLVCFRLKGSNKVNEAL LQRINSAKKIHLVPCHLRDKFVLRFAICSRTVESAHVQRAWEHIKELA ADVLRAERE |
| SEQ ID NO: 8 | AADC nucleotide sequence | ATGAACGCAAGTGAATTCCGAAGGAGAGGGAAGGAGATGGTGGA TTACATGGCCAACTACATGGAAGGCATTGAGGGACGCCAGGTCTA CCCTGACGTGGAGCCCGGGTACCTGCGGCCGCTGATCCCTGCCGCT GCCCCTCAGGAGCCAGACACGTTTGAGGACATCATCAACGACGTT GAGAAGATAATCATGCCTGGGGTGACGCACTGGCACAGCCCCTAC TTCTTCGCCTACTTCCCCACTGCCAGCTCGTACCCGGCCATGCTTGC GGACATGCTGTGCGGGGCCATTGGCTGCATCGGCTTCTCCTGGGCG GCAAGCCCAGCATGCACAGAGCTGGAGACTGTGATGATGGACTGG CTCGGGAAGATGCTGGAACTACCAAAGGCATTTTTGAATGAGAAA GCTGGAGAAGGGGGAGGAGTGATCCAGGGAAGTGCCAGTGAAGC CACCCTGGTGGCCCTGCTGGCCGCTCGGACCAAAGTGATCCATCG GCTGCAGGCAGCGTCCCCAGAGCTCACACAGGCCGCTATCATGGA GAAGCTGGTGGCTTACTCATCCGATCAGGCACACTCCTCAGTGGA AAGAGCTGGGTTAATTGGTGGAGTGAAATTAAAAGCCATCCCCTC AGATGGCAACTTCGCCATGCGTGCGTCTGCCCTGCAGGAAGCCCT GGAGAGAGACAAAGCGGCTGGCCTGATTCCTTTCTTTATGGTTGCC ACCCTGGGGACCACAACATGCTGCTCCTTTGACAATCTCTTAGAAG TCGGTCCTATCTGCAACAAGGAAGACATATGGCTGCACGTTGATG CAGCCTACGCAGGCAGTGCATTCATCTGCCCTGAGTTCCGGCACCT TCTGAATGGAGTGGAGTTTGCAGATTCATTCAACTTTAATCCCCAC AAATGGCTATTGGTGAATTTTGACTGTTCTGCCATGTGGGTGAAAA AGAGAACAGACTTAACGGGAGCCTTTAGACTGGACCCCACTTACC TGAAGCACAGCCATCAGGATTCAGGGCTTATCACTGACTACCGGC ATTGGCAGATACCACTGGGCAGAAGATTTCGCTCTTTGAAAATGTG GTTTGTATTTAGGATGTATGGAGTCAAAGGACTGCAGGCTTATATC CGCAAGCATGTCCAGCTGTCCCATGAGTTTGAGTCACTGGTGCGCC AGGATCCCCGCTTTGAAATCTGTGTGGAAGTCATTCTGGGGCTTGT CTGCTTTCGGCTAAAGGGTTCCAACAAAGTGAATGAAGCTCTTCTG CAAAGAATAAACAGTGCCAAAAAAATCCACTTGGTTCCATGTCAC CTCAGGGACAAGTTTGTCCTGCGCTTTGCCATCTGTTCTCGCACGG TGGAATCTGCCCATGTGCAGCGGGCCTGGGAACACATCAAAGAGC TGGCGGCCGACGTGCTGCGAGCAGAGAGGGAG |
| SEQ ID NO: 9 | GCH1 amino acid sequence | MEKGPVRAPAEKPRGARCSNGFPERDPPRPGPSRPAEKPPRPEAKSAQ PADGWKGERPRSEEDNELNLPNLAAAYSSILSSLGENPQRQGLLKTP WRAASAMQFFTKGYQETISDVLNDAIFDEDHDEMVIVKDIDMFSMCE HHLVPFVGKVHIGYLPNKQVLGLSKLARIVEIYSRRLQVQERLTKQIA VAITEALRPAGVGVVVEATHMCMVMRGVQKMNSKTVTSTMLGVFR EDPKTREEFLTLIRS |
| SEQ ID NO: 10 | GCH1 nucleotide sequence | ATGGAGAAGGGCCCTGTGCGGGCACCGGCGGAGAAGCCGCGGGG CGCCAGGTGCAGCAATGGGTTCCCCGAGCGGGATCCGCCGCGGCC CGGGCCCAGCAGGCCGGCGGAGAAGCCCCCGCGGCCCGAGGCCA AGAGCGCGCAGCCCGCGGACGGCTGGAAGGGCGAGCGGCCCCGC AGCGAGGAGGATAACGAGCTGAACCTCCCTAACCTGGCAGCCGCC TACTCGTCCATCCTGAGCTCGCTGGGCGAGAACCCCCAGCGGCAA GGGCTGCTCAAGACGCCCTGGAGGGCGGCCTCGGCCATGCAGTTC TTCACCAAGGGCTACCAGGAGACCATCTCAGATGTCCTAAACGAT GCTATATTTGATGAAGATCATGATGAGATGGTGATTGTGAAGGAC ATAGACATGTTTTCCATGTGTGAGCATCACTTGGTTCCATTTGTTG GAAAGGTCCATATTGGTTATCTTCCTAACAAGCAAGTCCTTGGCCT CAGCAAACTTGCGAGGATTGTAGAAATCTATAGTAGAAGACTACA AGTTCAGGAGCGCCTTACAAAACAAATTGCTGTAGCAATCACGGA AGCCTTGCGGCCTGCTGGAGTCGGGGTAGTGGTTGAAGCAACACA CATGTGTATGGTAATGCGAGGTGTACAGAAAATGAACAGCAAAAC TGTGACCAGCACAATGTTGGGTGTGTTCCGGGAGGATCCAAAGAC TCGGGAAGAGTTCCTGACTCTCATTAGGAGC |
| SEQ ID NO: 11 | TH amino acid sequence | MVKVPWFPRKVSELDKCHHLVTKFDPDLDLDHPGFSDQVYRQRRKL IAEIAFQYRHGDPIPRVEYTAEEIATWKEVYTTLKGLYATHACGEHLE AFALLERFSGYREDNIPQLEDVSRFLKERTGFQLRPVAGLLSARDFLA SLAFRVFQCTQYIRHASSPMHSPEPDCCHELLGHVPMLADRTFAQFSQ DIGLASLGASDEEIEKLSTLYWFTVEFGLCKQNGEVKAYGAGLLSSYG ELLHCLSEEPEIRAFDPEAAAVQPYQDQTYQSVYFVSESFSDAKDKLR SYASRIQRPFSVKFDPYTLAIDVLDSPQAVRRSLEGVQDELDTLAHAL SAIG |
| SEQ ID NO: 12 | TH nucleotide sequence | ATGGTGAAGGTCCCCTGGTTCCCAAGAAAAGTGTCAGAGCTGGAC AAGTGTCATCACCTGGTCACCAAGTTCGACCCTGACCTGGACTTGG ACCACCCGGGCTTCTCGGACCAGGTGTACCGCCAGCGCAGGAAGC TGATTGCTGAGATCGCCTTCCAGTACAGGCACGGCGACCCGATTCC CCGTGTGGAGTACACCGCCGAGGAGATTGCCACCTGGAAGGAGGT CTACACCACGCTGAAGGGCCTCTACGCCACGCACGCCTGCGGGGA GCACCTGGAGGCCTTTGCTTTGCTGGAGCGCTTCAGCGGCTACCGG GAAGACAATATCCCCCAGCTGGAGGACGTCTCCCGCTTCCTGAAG GAGCGCACGGGCTTCCAGCTGCGGCCTGTGGCCGGCCTGCTGTCC GCCCGGGACTTCCTGGCCAGCCTGGCCTTCCGCGTGTTCCAGTGCA CCCAGTATATCCGCCACGCGTCCTCGCCCATGCACTCCCCTGAGCC GGACTGCTGCCACGAGCTGCTGGGGCACGTGCCCATGCTGGCCGA CCGCACCTTCGCGCAGTTCTCGCAGGACATTGGCCTGGCGTCCCTG GGGGCCTCGGATGAGGAAATTGAGAAGCTGTCCACGCTGTACTGG TTCACGGTGGAGTTCGGGCTGTGTAAGCAGAACGGGGAGGTGAAG GCCTATGGTGCCGGGCTGCTGTCCTCCTACGGGGAGCTCCTGCACT GCCTGTCTGAGGAGCCTGAGATTCGGGCCTTCGACCCTGAGGCTGC GGCCGTGCAGCCCTACCAAGACCAGACGTACCAGTCAGTCTACTT CGTGTCTGAGAGCTTCAGTGACGCCAAGGACAAGCTCAGGAGCTA TGCCTCACGCATCCAGCGCCCCTTCTCCGTGAAGTTCGACCCGTAC ACGCTGGCCATCGACGTGCTGGACAGCCCCCAGGCCGTGCGGCGC TCCCTGGAGGGTGTCCAGGATGAGCTGGACACCCTTGCCCATGCG CTGAGTGCCATTGGA |
| SEQ ID NO: 13 | P2A amino acid sequence | GSGATNFSLLKQAGDVEENPGP |
| SEQ ID NO: 14 | P2A nucleotide sequence | GGAAGCGGAGCTACTAACTTCAGCCTGCTGAAGCAGGCTGGCGAC GTGGAGGAGAACCCTGGACCT |
| SEQ ID NO: 15 | F2A amino acid sequence | GSGVKQTLNFDLLKLAGDVESNPGP |
| SEQ ID NO: 16 | F2A nucleotide sequence | GGAAGCGGAGTGAAACAGACTTTGAATTTTGACCTTCTCAAGTTG GCGGGAGACGTGGAGTCCAACCCTGGACCT |
| SEQ ID NO: 17 | GS15 amino acid sequence | GGGGSGGGGSGGGGS |
| SEQ ID NO: 18 | GS15 nucleotide sequence | GGAGGCGGCGGCTCTGGTGGAGGCGGCAGCGGCGGCGGCGGTTCT |

The nucleotide sequences of AADC-P2A-GCH1-P2A-TH (SEQ ID NO: 19), TH-GS15-GCH1-P2A-AADC (SEQ ID NO: 20), and TH-F2A-GCH1-P2A-AADC (SEQ ID NO: 21) (wherein the TH is a truncated form of TH); a synapsin I promoter (SEQ ID NO: 2) and a CMV promoter (SEQ ID NO: 3) were artificially synthesized. The synthesized genes were cloned into pAAV-MCS-CBH-SV40 (the nucleotide sequence is shown in SEQ ID NO: 22, and the plasmid profile is shown in FIG. 2), an adeno-associated virus backbone developed by the applicant, in a recombinant linking manner to prepare recombinant adeno-associated virus vectors (rAAVs). The following vectors were obtained, with the structural schematic diagram as shown in FIG. 1.

### CBH-DGT vector:

AADC-P2A-GCH1-P2A-TH was digested with AgeI and SalI, ligated and cloned into the site between CBH and SV40 of the adeno-associated virus backbone vector pAAV-MCS-CBH-SV40. The resulting vector was designated as CBH-DGT vector.

### CBH-TFGPD vector:

TH-F2A-GCH1-P2A-AADC was digested with AgeI and SalI, ligated and cloned into the site between CBH and SV40 of the adeno-associated virus backbone vector pAAV-MCS-CBH-SV40. The resulting vector was designated as CBH-TFGPD vector.

### CBH-TGPD vector:

TH-GS15-GCH1-P2A-AADC was digested with AgeI and SalI, ligated and cloned into the site between CBH and SV40 of the adeno-associated virus backbone vector pAAV-MCS-CBH-SV40. The resulting vector was designated as CBH-TGPD vector.

### CMV-TGPD vector:

The CBH promoter in the CBH-TGPD vector was replaced with a CMV promoter. The resulting vector was designated as CMV-TGPD vector.

### synapsin-TGPD vector:

The CBH promoter in the CBH-TGPD vector was replaced with a Synapsin I promoter. The resulting vector was designated as synapsin-TGPD vector.

### CBH-EGFP vector:

The EGFP sequence was cloned and substituted for the AADC-P2A-GCH1-P2A-TH sequence in the CBH-DGT vector. The resulting vector was designated as CBH-EGFP vector.

### Example 2. Packaging and determination of AAV virus particles

The vector containing the gene of interest obtained in Example 1 (CBH-DGT, CBH-TGPD, CBH-TFGPD, CMV-TGPD, synapsin-TGPD, or CBH-EGFP), a packaging plasmid with different serotypes (KL-pAAV5(SEQ ID NO: 24), KL-pAAV9(SEQ ID NO: 25), KL-pAAVPHP.eB(SEQ ID NO: 26), KL-pAAVPHP.S(SEQ ID NO: 27), or KL-pAAVPHP.B(SEQ ID NO: 28)) and a helper plasmid (KL-pAAV-Helper, nucleotide sequence of SEQ ID NO: 29) were co-transfected into 293T cells (purchased from American Type Culture Collection (ATCC), deposit number CRL-3216), and the AAVs were packaged using the 293T system. The transfection method was PEI cationic polymer (PEI-Max transfection reagent, purchased from Polysciences, Cat. No.: 24765-1) mediated transient transfection of eukaryotic cells. The transfection procedure was carried out according to the standardized procedure recommended by the manufacturer, and the transfection scale was 15 cm² cell culture dishes.

72 hours after the end of transfection, the cell supernatant was collected together with the cells into a 50 mL centrifuge tube. Cells and supernatants were separated by centrifugation using a benchtop centrifuge at 4200 rpm for 10 minutes at room temperature. Then the supernatant was transfer into a new 50 mL centrifuge tube. MgCl₂ and ribozyme was added and mixed well for later use. The remaining cells were added with ribozyme and cell lysate, allowed to stand at room temperature for 1 h, and then centrifuged at 10000g at room temperature for 10 mins. The supernatant was transferred into the cell supernatant obtained in the previous step, mixed well, and finally subjected to affinity purification and concentration to obtain AAV virus particles of different serotypes (AAV9-CBH-DGT, AAV9-CBH-TGPD, AAV9-CBH-TFGPD, AAV9-CMV-TGPD, AAV9-synapsin-TGPD, AAV5-CBH-TGPD, AAVPHP.eB-CBH-TGPD, AAVPHP.S-CBH-TGPD, AAVPHP.B-CBH-TGPD, and AAV9-CBH-EGFP).

Physical titers of the AAVs were calculated from quantitative PCR results and AAV standard as a reference using methods well known in the art (see Table 2). The primer probe sequences used for quantitative PCR were:
CMV-forward primer:
   ACTTTCCATTGACGTCAATGGGTGG (SEQ ID NO: 30)
CMV Probe:
   5'- CAAGTGTATCATATGCCAAGTACGCCCCC -3' (SEQ ID NO: 31)
CMV-reverse primer:
   AGGTCATGTACTGGGCATAATGC (SEQ ID NO: 32)
wherein the 5' end of the CMV probe was labeled with a FAM fluorescent group and the 3' was labeled with a BHQ1 fluorescent group.

**Table 2. Physical titers of different AAV virus particles**

| Virus name | Viral titer (vg/ml) |
|---|---|
| AAV9-CBH-EGFP | 9.05E+12 |
| AAV9-CBH-DGT | 8.96E+ 12 |
| AAV9-CBH-TGPD | 1.91E+13 |
| AAV9-CBH-TFGPD | 1.34E+13 |
| AAV9-CMV-TGPD | 4.28E+12 |
| AAV9-synapsin-TGPD | 1.27E+13 |
| AAV5-CBH-TGPD | 3.47E+ 13 |
| AAVPHP.eB-CBH-TGPD | 8.44E+12 |
| AAVPHP.S-CBH-TGPD | 7.19E+12 |
| AAVPHP.B-CBH-TGPD | 3.88E+12 |

The results as shown in Table 2 indicated that the different AAV vectors in Example 1 can be effectively packaged by capsid proteins with various AAV serotypes, thereby obtaining AAV virus particles with high physical titers.

### Example 3. Determination of intracellular expression of proteins of interest in 293T cells after being infected with AAV virus particles

293T cells were infected with the packaged AAV virus particle AAV9-CBH-TFGPD with the same multiplicity of infection (MOI). After the infection, the cells were lysed to produce a cell lysate, which was then used for Western blot experiments. After an SDS-PAGE electrophoresis, the proteins of interest were transferred to a PVDF membrane, and the expressions of the proteins of interest were detected with anti-GCH1, anti-TH, and anti-AADC antibodies as primary antibodies. The anti-GAPDH antibody was used as an internal reference primary antibody for detecting the expression of the internal reference protein. The experimental results (see FIG. 3) showed that the AAV virus particle AAV9-CBH-TFGPD was able to efficiently express the proteins of interest, GCH1, TH1 and AADC after being transducted into cells in vitro.

### Example 4. Determination of dopamine synthesis levels mediated by AAV vectors in vitro

293T cells were infected with the packaged AAV virus particles at the same MOI, and the cell supernatant was collected 72 hours later, and the concentration of dopamine and its metabolites in the cell supernatant was detected by high-performance liquid chromatography. The experimental results (see Table 3 and FIG. 4) showed that after the in vitro transduction of cells with AAV9-CBH-TFGPD, the intracellular dopamine synthesis was significantly increased, and dopamine metabolites 3,4-dihydroxyphenylacetic acid and homovanillic acid were significantly increased, demonstrating that AAV9-CBH-TFGPD can mediate high-efficiency dopamine synthesis in cells under in vitro conditions.

**Table 3. Determination of in vitro dopamine synthesis levels and its metabolites mediated by AAV vectors**

| **AAV vector** | **MOI** | **3,4-Dihydroxyphenylacetic acid** | **Homovanillic acid** | **Dopamine** |
|---|---|---|---|---|
| | | **Concentration ng/mL** | | |
| AAV9-CBH-EGFP | | 75.8 | 186.7 | 37.1 |
| | | 76.1 | 174.6 | 49.1 |
| | 5.00E+05 | 76.2 | 175.8 | 49.2 |
| AAV9-CBH-TFGPD | | 340.3 | 845.9 | 4394.0 |
| | | 305.7 | 807.6 | 4448.5 |
| | | 318.4 | 768.2 | 4650.8 |

### Example 5. Determination of in vivo dopamine synthesis efficiency mediated by various AAV vectors

AAV virus particles packaging various AAV vectors were delivered into the brain of wild-type C57/B6 mice at the same dose by brain stereotaxic injection to infect nerve cells in the brain of mice. The brains were separated two weeks after injection. 200 µL of a solution containing 0.2 mM HClO₄ and 1 mM cysteine was added to every 30 mg of brain tissue, and the mixture was homogenized into a cell lysate. The supernatant was collected by centrifugation at 4°C and 12000 rpm for 5 minutes, and the concentration of dopamine and its metabolites in the cell lysate was determined by high-performance liquid chromatography. The experimental results (see Table 4 and FIG. 5) showed that although AAV9-CBH-DGT did not show a significant difference in the level of dopamine synthesis, the metabolites of dopamine, 3,4-dihydroxyphenylacetic acid and homovanillic acid, were significantly increased compared with the control group, indicating that dopamine was rapidly metabolized after dopamine synthesis. These results indicate that various AAV vectors can efficiently mediate dopamine synthesis after infecting nerve cells in vivo, when compared with the control group.

**Table 4. In vivo dopamine synthesis and the content of its metabolites mediated by various AAV vectors**

| **AAV vector** | **3,4-Dihydroxyphenylacetic acid** | **Homovanillic acid** | **Dopamine** |
|---|---|---|---|
| | **ng/mg** | **ng/mg** | **ng/mg** |
| AAV9-CBH-EGFP | 0.30 | 0.13 | 0.21 |
| | 0.37 | 0.13 | 0.27 |
| | 0.35 | 0.10 | 0.27 |
| AAV9-CBH-DGT | 0.91 | 1.99 | 0.32 |
| | 0.63 | 1.15 | 0.39 |
| | 0.75 | 1.25 | 0.40 |
| AAV9-CBH-TGPD | 3.14 | 5.37 | 0.81 |
| | 2.02 | 3.64 | 0.74 |
| | 4.71 | 5.79 | 1.64 |

### Example 6. Determination of in vivo dopamine synthesis efficiency mediated by AAV vectors with different serotypes

AAV vectors with different serotypes were delivered into the brain of wild-type C57/B6 mice at the same dose by brain stereotaxic injection to infect nerve cells in the brain of mice. The brains were separated two weeks after injection. 200 µL of a solution containing 0.2mM HClO₄ and 1 mM cysteine was added to every 30 mg of brain tissue, and the mixture was homogenized into a cell lysate. The supernatant was collected by centrifugation at 4°C and 12000 rpm for 5 minutes, and the concentration of dopamine and its metabolites in the cell lysate was determined by high-performance liquid chromatography. The experimental results (see Table 5 and FIG. 6) showd that all the AAV vectors with AAV9, AAV5, AAVPHP.eB, AAVPHP.S, and AAVPHP.B serotypes exhibited a significant increase in the level of dopamine synthesis, and the increase in dopamine metabolites 3,4-dihydroxyphenylacetic acid and homovanillic acid was more pronounced than that of the control group, indicating that dopamine is rapidly metabolized after dopamine synthesis. Therefore, the experimental results showed that AAV vectors with various serotypes can efficiently synthesize dopamine after infecting nerve cells in vivo, when compared with the control group.

**Table 5. In vivo dopamine synthesis and the content of its metabolites mediated by AAV vectors with different serotypes**

| **AAV vector** | **Dopamine** | **3,4-Dihydroxyphenylacetic acid** | **Homovanillic acid** |
|---|---|---|---|
| | **ng/mg** | **ng/mg** | **ng/mg** |
| AAV9-CBH-EGFP | 0.12 | <min | 0.08 |
| | 0.14 | <min | 0.07 |
| | 0.19 | <min | 0.09 |
| AAV9-CBH-TGPD | 1.37 | 3.84 | 4.67 |
| | 0.86 | 2.05 | 2.87 |
| | 1.41 | 3.67 | 4.96 |
| AAV5-CBH-TGPD | 0.35 | 0.11 | 0.87 |
| | 0.41 | 0.75 | 1.13 |
| | 0.46 | 0.82 | 1.26 |
| AAVPHP.eB-CBH-TGPD | 0.54 | 0.11 | 1.17 |
| | 0.39 | 0.12 | 1.36 |
| | 0.74 | 1.32 | 3.97 |
| AAVPHP.S-CBH-TGPD | 0.33 | 0.08 | 0.56 |
| | 0.57 | 0.68 | 2.00 |
| | 0.32 | 0.34 | 1.41 |
| AAVPHP.B-CBH-TGPD | 1.04 | 1.91 | 3.98 |
| | 0.41 | 0.86 | 1.60 |
| | 0.82 | 1.83 | 4.65 |

### Example 7. Determination of in vivo dopamine synthesis efficiency mediated by AAV vectors using various promoters

Virus particles packaging AAV vectors using different promoters were delivered into the brain of wild-type C57/B6 mice at the same dose by brain stereotaxic injection to infect nerve cells in the brain of mice. The brains were separated two weeks after injection. 200 µL of a solution containing 0.2 mM HClO₄ and 1 mM cysteine was added to every 30 mg of brain tissue, and the mixture was homogenized into a cell lysate. The supernatant was collected by centrifugation at 4°C and 12000rpm for 5 minutes, and the concentration of dopamine in the cell lysate was determined by high-performance liquid chromatography. The experimental results (see Table 6 and FIG. 7) showed that AAV vectors using various promoters can efficiently synthesize dopamine after infecting nerve cells in vivo, when compared with the control group.

**Table 6. In vivo dopamine synthesis levels mediated by AAV vectors using various promoters**

| **AAV vector** | **Dopamine** |
|---|---|
| | **ng/mg** |
| AAV9-CBH-EGFP | 0.20 |
| | 0.23 |
| | 0.16 |
| AAV9-CBH-TGPD | 1.02 |
| | 1.01 |
| | 0.96 |
| AAV9-Syn-TGPD | 0.50 |
| | 0.89 |
| | 0.50 |
| AAV9-CMV-TGPD | 0.49 |
| | 0.36 |
| | 0.38 |

### Example 8. Determination of in vivo dopamine synthesis efficiency mediated by AAV vectors using various linkers

AAV virus particles packaging AAV vectors using different linkers were delivered into the brain of wild-type C57/B6 mice at the same dose by brain stereotaxic injection to infect nerve cells in the brain of mice. The brains were separated two weeks after injection. 200 µL of a solution containing 0.2 mM HClO₄ and 1 mM cysteine was added to every 30 mg of brain tissue, and the mixture was homogenized into a cell lysate. The supernatant was collected by centrifugation at 4°C and 12000 rpm for 5 minutes, and the concentration of dopamine in the cell lysate was determined by high-performance liquid chromatography. The experimental results (see Table 7 and FIG. 8) showed that AAV vectors using various linkers can efficiently synthesize dopamine after infecting nerve cells in vivo, when compared with the control group.

**Table 7. In vivo dopamine synthesis levels mediated by AAV vectors using various linkers**

| **AAV vector** | **Dopamine concentration** |
|---|---|
| | **ng/mg** |
| AAV9-CBH-EGFP | 0.22 |
| | 0.25 |
| | 0.2 |
| AAV9-CBH-TGPD | 1.37 |
| | 0.86 |
| | 1.39 |
| AAV9-CBH-TFGPD | 1.26 |
| | 0.85 |
| | 0.78 |

### Example 9. Evaluation of the efficacy of AAV9-CBH-TGPD in a rat model of Parkinson's disease

A rat model of unilateral Parkinson's disease was developed by brain stereotaxic injection of 6-hydroxydopamine (6-OHDA) into the unilateral medial forebrain bundle (MFB) brain region of SD rats. SD rats were injected with apomorphine hydrochloride (APO) (1 mg/kg) intraperitoneally at 4 weeks after 6-OHDA injection. The rats were induced to rotate to the healthy side, and the modeling quality of the animals was evaluated by the rotation speed.

After the screening of suitable rat models of Parkinson's disease, the modeled animals were divided into groups according to the APO induction results of the animals, and AAV9-CBH-EGFP virus particles and various doses of AAV9-CBH-TGPD virus particles were administered to the striatum of the injured side by brain stereotaxic injection (see Table 8). Apomorphine hydrochloride (1 mg/kg) was administered intraperitoneally at 4, 5, 6, 7, 8, 9, 10, 11, 12, 13, 15, 17, 19, 21 and 23 weeks after administration of virus particles, respectively, and the rotations of animals 5 - 10 min, 15 - 20 min, and 25 - 30 min after administration of the induction drug were counted respectively. Compared with the control group, the number of rotations after APO induction decreased significantly in each dosing group of AAV9-CBH-TGPD, and the dosage of AAV9-CBH-TGPD showed a negative correlation with the number of rotations after induction (see FIG. 9). That is, the higher the dosage, the lower the number of rotations after APO induction, demonstrating that AAV9-CBH-TGPD had an excellent therapeutic effect in the rat model of Parkinson's disease.

**Table 8. Animal grouping and dosing**

| **Group** | **Number of animals/sex** | **AAV vector** | **Injection site/volume** | **Dosage of test substance** (vg/striatum) |
|---|---|---|---|---|
| 1 | 7 male rats with Parkinson's disease | AAV9-CBH-EGFP | Striatum (4 sites * 5µL) | 3.2E10 |
| 2 | 7 male rats with Parkinson's disease | AAV9-CBH-TGPD | Striatum (4 sites * 5 µL) | 2.0E9 |
| 3 | 7 male rats with Parkinson's disease | AAV9-CBH-TGPD | Striatum (4 sites * 5 µL) | 8.0E9 |
| 4 | 7 male rats with Parkinson's disease | AAV9-CBH-TGPD | Striatum (4 sites * 5 µL) | 3.2E10 |

The best therapeutic effect of lentiviral vector drug, Prosavin (Mimoun Azzouz, et al. The Journal of Neuroscience, 2002, 22(23):10302-10312), for the treatment of Parkinson's disease, was that the rotational behavior induced by apomorphine was reduced from 8 r/min in the control group to 5 r/min in the treatment group (the effect was similar to clinical trial data). The AAV-CBH-TGPD of the present invention has achieved an effect close to functional cure, which reduced apomorphine-induced rotational behavior from 10 r/min in the control group to close to 1 r/min in the treatment group.

### Example 10. Safety testing of AAV virus particles and buffer system

Using the method of Example 2, the vector CBH-TGPD obtained in Example 1 was packaged with AAV9, AAV2, and AAV5 serotype packaging plasmids, respectively, to obtain AAV virus particles with various serotypes. Wherein the AAV9 and AAV5 packaging plasmids were identical to those in Example 2, and the sequence of AAV2 packaging plasmid was that shown as SEQ ID NO: 48.

The AAV virus particles were mixed with the buffer system at a total dose of 8.0E+9vg to prepare the composition. The buffer system was 10mM sodium phosphate buffer (NaH₂PO₄&Na₂HPO₄), 150 mM sodium chloride, and 0.001% F68, pH 7.4, in a total volume of 10 microliters. The buffer system, or the compositions of AAV virus particles and the buffer system, was delivered into the brain of wild-type C57/B6 mice by brain stereotaxic injection, respectively. The neurotoxicity of the compositions comprising the buffer system and the AAVs with different serotypes was observed.

Two weeks after injection, mice were subjected to Nissl staining to determine neurotoxicity. The brains of mice were taken, and the striatum region was subjected to paraffin sections. The samples were stained according to the instructions of the Nissl staining kit (Sangon Biotech (Shanghai) Co., Ltd., E607316-0100), and then imaged under an optical microscope. The results of the Nissl staining experiment are shown in FIG. 10. It was showed that compared with the buffer system group, there was no abnormality in the number, shape, distribution, and the like, of neurons in mice injected with the buffer system + AAV9 composition, the buffer system + AAV2 composition, and the buffer system + AAV5 composition, indicating that none of the AAV virus particles of the present application had neurotoxicity, and the composition with the buffer system also met the safety requirements.

Although the present disclosure has been described with reference to specific embodiments thereof, it will be understood by those skilled in the art that various changes may be made and equivalents may be substituted without departing from the true spirit and scope of the present invention. In addition, many modifications can be made to adapt the particular situation, material, composition, method, the steps of method to the purpose, spirit and scope of the present invention. All such modifications are intended to be within the scope of the present invention.

## Claims

1. An adeno-associated virus (AAV) vector comprising nucleic acid sequences encoding tyrosine hydroxylase (TH), GTP-cyclohydrolase 1 (GCH1) and aromatic amino acid decarboxylase (AADC), wherein the nucleic acid sequences encoding the TH, the GCH1 and/or the AADC are in-frame linked by a nucleic acid sequence encoding a linker, wherein any one or more of the TH, the GCH1 and the AADC is a truncated form of wild type or a functional variant thereof and has the catalytic activity required in the dopamine synthesis pathway.

2. The AAV vector of claim 1, wherein the TH is a truncated form of wild-type TH or a functional variant thereof and has the activity of catalyzing the synthesis of levodopa from tyrosine; preferably, the TH comprises 330 to 390 amino acid residues.

3. The AAV vector of claim 1 or 2, wherein the TH comprises an amino acid sequence having at least 75%, 80%, 85%, 90%, 95%, 96%, 97%, 98%, 99%, or 100% identity to SEQ ID NO: 11.

4. The AAV vector of any one of claims 1-3, wherein the GCH1 comprises an amino acid sequence having at least 75%, 80%, 85%, 90%, 95%, 96%, 97%, 98%, 99%, or 100% identity to SEQ ID NO: 9.

5. The AAV vector of any one of claims 1-4, wherein the AADC comprises an amino acid sequence having at least 75%, 80%, 85%, 90%, 95%, 96%, 97%, 98%, 99%, or 100% identity to SEQ ID NO: 7.

6. The AAV vector of any one of claims 1-5, wherein the linker comprises a fusion linker, a 2A peptide linker, and/or an internal ribosome entry site (IRES).

7. The AAV vector of any one of claim 6, wherein the fusion linker is a peptide linker; preferably, the peptide linker is selected from (GGS)n, (GGGS)n or (GGGGS)n, wherein n is an integer from 1 to 5.

8. The AAV vector of claim 7, wherein the fusion linker is (G4S)3.

9. The AAV vector of claim 6, wherein the 2A peptide linker comprises a 2A peptide selected from foot-and-mouth disease virus 2A peptide (F2A), porcine teschovirus 2A peptide (P2A), Thosea asign virus 2A peptide (T2A), and/or equine rhinitis virus 2A peptide (E2A).

10. The AAV vector of claim 9, wherein the N-terminus and/or C-terminus, preferably the N-terminus, of the 2A peptide linker further comprises a GSG amino acid sequence.

11. The AAV vector of claim 10, wherein the 2A peptide linker comprises an amino acid sequence having at least 75%, 80%, 85%, 90%, 95%, 96%, 97%, 98%, 99%, or 100% identity to SEQ ID NO: 13 or SEQ ID NO: 15.

12. The AAV vector of any one of claims 1-11, wherein the nucleic acid sequences encoding the TH, the GCH1, and the AADC are arranged from upstream to downstream in an order selected from any one of:
(1) TH, GCH1, AADC;
(2) AADC, GCH1, TH;
(3) AADC, TH, GCH1;
(4) GCH1, TH, AADC;
(5) GCH1, AADC, TH; or
(6) TH, AADC, GCH1.

13. The AAV vector of claim 12, wherein the nucleic acid sequences encoding the TH, the GCH1, and the AADC are linked from upstream to downstream in a manner selected from any one of:
1) TH-F2A-GCH1-P2A-AADC;
2) GCH1-P2A-TH-P2A-AADC;
3) GCH1-P2A-AADC-P2A-TH;
4) AADC-E2A-GCH1-(G4S)3-TH;
5) GCH1-F2A-TH-F2A-AADC;
6) AADC-(G4S)3-TH-T2A-GCH1;
7) AADC-(G4S)4-TH-T2A-GCH1;
8) AADC-P2A-GCH1-P2A-TH;
9) AADC-T2A-GCH1-(G4S)3-TH;
10) GCH1-F2A-TH-P2A-AADC;
11) GCH1-F2A-AADC-F2A-TH;
12) AADC-(G4S)3-GCH1-F2A-TH;
13) GCH1-E2A-TH-T2A-AADC;
14) GCH1-F2A-AADC-P2A-TH;
15) AADC-(G4S)3-GCH1-P2A-TH;
16) AADC-(G4S)5-GCH1-P2A-TH;
17) TH-(G4S)3-GCH1-P2A-AADC;
18) GCH1-T2A-AADC-E2A-TH;
19) AADC-(G4S)3-GCH1-T2A-TH;
20) GCH1-E2A-TH-P2A-AADC;
21) AADC-(G4S)3-GCH1-E2A-TH;
22) GCH1-E2A-AADC-P2A-TH;
23) AADC-(G4S)3-GCH1-(G4S)3-TH;
24) GCH1-T2A-TH-E2A-AADC;
25) GCH1-(G4S)3-TH-T2A-AADC;
26) GCH1-(G4S)2-TH-T2A-AADC;
27) GCH1-P2A-AADC-T2A-TH;
28) TH-P2A-GCH1-P2A-AADC;
29) TH-(G4S)3-GCH1-T2A-AADC;
30) GCH1-(G4S)3-TH-E2A-AADC;
31) GCH1-T2A-TH-(G4S)3-AADC;
32) TH-P2A-GCH1-F2A-AADC;
33) GCH1-F2A-TH-(G4S)3-AADC;
34) TH-F2A-GCH1-F2A-AADC;
35) TH-(G4S)3-GCH1-E2A-AADC;
36) TH-G4S-GCH1-E2A-AADC;
37) GCH1-P2A-TH-(G4S)3-AADC;
38) AADC-F2A-GCH1-P2A-TH;
39) TH-T2A-GCH1-(G4S)3-AADC;
40) GCH1-E2A-TH-(G4S)3-AADC;
41) TH-E2A-GCH1-(G4S)3-AADC;
42) TH-T2A-GCH1-E2A-AADC;
43) AADC-F2A-GCH1-F2A-TH;
44) TH-F2A-GCH1-(G4S)3-AADC;
45) GCH1-(G4S)3-TH-(G4S)3-AADC;
46) TH-(G4S)3-GCH1-F2A-AADC;
47) TH-E2A-GCH1-T2A-AADC;
48) AADC-P2A-TH-(G4S)3-GCH1;
49) TH-(G4S)3-GCH1-(G4S)3-AADC;
50) AADC-F2A-TH-(G4S)3-GCH1;
51) TH-F2A-AADC-F2A-GCH1;
52) TH-(G4S)3-AADC-T2A-GCH1;
53) TH-(G4S)3-AADC-E2A-GCH1;
54) AADC-P2A-GCH1-(G4S)3-TH;
55) TH-F2A-AADC-(G4S)3-GCH1;
56) AADC-F2A-GCH1-(G4S)3-TH;
57) TH-P2A-AADC-F2A-GCH1;
58) TH-P2A-AADC-(G4S)3-GCH1;
59) GCH1-(G4S)3-TH-P2A-AADC;
60) TH-T2A-AADC-(G4S)3-GCH1;
61) TH-T2A-AADC-E2A-GCH1;
62) GCH1-(G4S)3-TH-F2A-AADC;
63) TH-E2A-AADC-(G4S)3-GCH1;
64) AADC-E2A-GCH1-T2A-TH;
65) GCH1-P2A-TH-F2A-AADC;
66) TH-(G4S)3-AADC-(G4S)3-GCH1;
67) TH-E2A-AADC-T2A-GCH1;
68) GCH1-P2A-AADC-F2A-TH;
69) AADC-T2A-TH-(G4S)3-GCH1;
70) AADC-T2A-GCH1-E2A-TH;
71) TH-F2A-AADC-P2A-GCH1;
72) AADC-E2A-TH-(G4S)3-GCH1;
73) AADC-P2A-GCH1-F2A-TH;
74) TH-(G4S)3-AADC-F2A-GCH1;
75) AADC-P2A-TH-F2A-GCH1;
76) AADC-(G4S)3-TH-P2A-GCH1;
77) AADC-T2A-TH-E2A-GCH1;
78) AADC-(G4S)3-TH-E2A-GCH1;
79) AADC-E2A-TH-T2A-GCH1; or
80) AADC-(G4S)3-TH-F2A-GCH1.

14. The AAV vector of claim 13, wherein the AAV vector comprises the nucleic acid sequence set forth in any one of SEQ ID NOs: 19-21.

15. The AAV vector of any one of claims 1-14, further comprising a promoter operably linked to the nucleic acid sequences encoding the TH, the GCH1, and the AADC; preferably, wherein the promoter is selected from a CBH promoter, a chimeric Synapsin I promoter, a CMV promoter, a CAG promoter, a CAGG promoter or a CASI promoter.

16. The AAV vector of claim 15, wherein the promoter comprises a nucleic acid sequence having at least 75%, 80%, 85%, 90%, 95%, 96%, 97%, 98%, 99%, or 100% identity to SEQ ID NOs: 1-6; preferably, the promoter comprises the nucleic acid sequence set forth in any one of SEQ ID NOs: 1-3; more preferably, the promoter comprises the nucleic acid sequence set forth in SEQ ID NO: 1.

17. The AAV vector of any one of claims 1-16, wherein the AAV vector further comprises one or more elements selected from a KOZAK sequence, a polyadenylation signal of an SV40 virus (SV40 poly A), and an inverted terminal repeat (ITR).

18. An AAV virus particle comprising the AAV vector of any one of claims 1-17, and a capsid protein.

19. The AAV virus particle of claim 18, wherein the capsid protein is derived from an AAV selected from the following serotypes: AAV5, AAV9, AAVPHP.eB, AAVPHP.S or AAVPHP.B.

20. The AAV virus particle of claim 19, wherein the capsid protein comprises an amino acid sequence having at least 75%, 80%, 85%, 90%, 95%, 96%, 97%, 98%, 99%, or 100% identity to any one of SEQ ID NOs: 33-37; preferably, the capsid protein comprises an amino acid sequence selected from SEQ ID NO: 34 or 37; more preferably, the capsid protein comprises the amino acid sequence set forth in SEQ ID NO: 34.

21. A composition comprising the AAV vector of any one of claims 1-17 and a packaging plasmid encoding a capsid protein, and optionally a helper plasmid.

22. The composition of claim 21, wherein the packaging plasmid further comprises a nucleic acid sequence encoding a Rep protein, wherein the Rep protein is derived from an AAV of AAV2 or AAVPHP.B serotype.

23. The composition of claim 22, wherein the Rep protein comprises an amino acid sequence having at least 75%, 80%, 85%, 90%, 95%, 96%, 97%, 98%, 99%, or 100% identity to SEQ ID NO: 45 or SEQ ID NO: 46.

24. The composition of any one of claims 21-23, wherein the packaging plasmid comprises a nucleic acid sequence having at least 75%, 80%, 85%, 90%, 95%, 96%, 97%, 98%, 99%, or 100% identity to any one of SEQ ID NOs: 24-28 and 48; preferably, the packaging plasmid comprises a nucleic acid sequence selected from SEQ ID NO: 25 or 28; more preferably, the packaging plasmid comprises the nucleic acid sequence set forth in SEQ ID NO: 25.

25. The composition of any one of claims 21-24, wherein the helper plasmid comprises a nucleic acid sequence having at least 75%, 80%, 85%, 90%, 95%, 96%, 97%, 98%, 99%, or 100% identity to SEQ ID NO: 29.

26. The composition of any one of claims 21-25, further comprising a pharmaceutically acceptable carrier, diluent, or excipient.

27. A composition comprising the AAV virus particle of any one of claims 18-20; optionally, the composition further comprises a pharmaceutically acceptable carrier, diluent, or excipient.

28. The composition of claim 26 or 27 comprising a phosphate buffer, sodium chloride, and a surfactant, and preferably, the surfactant is poloxamer 188.

29. A cell comprising the AAV vector of any one of claims 1-17, the AAV virus particle of any one of claims 18-20, or the composition of any one of claims 21-28.

30. The cell of claim 29, wherein the cell is a nerve cell, as distinguished by function, the nerve cell is selected from: an inhibitory neuron (preferably, said cell is a medium spiny neuron, an interneuron), an excitatory neuron (preferably, said cell is a dopaminergic neuron), a microglia, an astrocyte, an oligodendrocyte, or a Schwann cell; as distinguished by brain region, the cell is a midbrain striatal neuron and a glial cell.

31. Use of the AAV vector of any one of claims 1-17, the AAV virus particle of any one of claims 18-20, the composition of any one of claims 21-28, or the cell of claim 29 or 30 in the manufacture of a medicament for preventing and/or treating a disease caused by dopamine deficiency in a subject.

32. The use of claim 31, wherein the disease is Parkinson's disease.
